(19) **Europäisches Patentamt / European Patent Office / Office européen des brevets**

(11) **EP 1 455 722 B2**

(12) **NEW EUROPEAN PATENT SPECIFICATION**
After opposition procedure

(45) Date of publication and mention
of the opposition decision:
**28.12.2011 Bulletin 2011/52**

(45) Mention of the grant of the patent:
**16.01.2008 Bulletin 2008/03**

(21) Application number: **02778508.8**

(22) Date of filing: **09.10.2002**

(51) Int Cl.:
***A61F 13/84*** *(2006.01)*    ***A61F 13/49*** *(2006.01)*

(86) International application number:
**PCT/US2002/032487**

(87) International publication number:
**WO 2003/057122 (17.07.2003 Gazette 2003/29)**

(54) **SYSTEM FOR SKIN HEALTH OF ABSORBENT ARTICLE WEARERS**

SYSTEM ZUR HAUTPFLEGE VON BENUTZERN VON ABSORBIERENDEN ARTIKELN

SYSTEME POUR AMELIORER LA SANTE DERMATOLOGIQUE DES UTILISATEURS D'ARTICLES ABSORBANTS

(84) Designated Contracting States:
**DE FR GB IT NL SE**

(30) Priority: **22.12.2001 US 28338**

(43) Date of publication of application:
**15.09.2004 Bulletin 2004/38**

(73) Proprietor: **KIMBERLY-CLARK WORLDWIDE, INC.**
**Neenah, WI 54956 (US)**

(72) Inventors:
• **BROCK, Earl, David**
 **Appleton, WI 54915 (US)**
• **KRZYSIK, Duane, Gerard**
 **Appleton, WI 54911 (US)**
• **SOLBERG, Rhonda, Sue**
 **Appleton, WI 54911 (US)**

• **MILLER, Stephen, Lawrence**
 **Appleton, WI 54915 (US)**
• **TYRRELL, David, John**
 **Appleton, WI 54911 (US)**

(74) Representative: **Beacham, Annabel Rose et al**
 **Dehns**
 **St Bride's House**
 **10 Salisbury Square**
 **London**
 **EC4Y 8JD (GB)**

(56) References cited:
 **EP-A- 0 350 275      WO-A-00/64501**
 **WO-A-00/64502      WO-A-00/64503**
 **WO-A-01/72262      WO-A1-01/00253**
 **WO-A2-02/051456      CH-A- 423 673**
 **US-A- 4 790 840      US-A- 5 141 803**
 **US-A- 5 569 330**

**Description**

**Background of the Invention**

The present invention is directed to a system for improving the skin health of wearers of absorbent articles. Absorbent articles are designed to receive and contain fluid and solid wastes, discharges and exudates. While modern day absorbent articles are capable of performing their intended function excellently, coverage (and possibly occlusion) of the wearer's skin cannot be avoided. Consequently, consideration has been given to maintaining and even improving the health of the skin covered by absorbent articles. With the present invention, absorbent articles having a non-aqueous composition are used in conjunction with substrates (such as wipes) having a hydrophilic composition to provide benefits not achievable through the use of such articles and such substrates independently.

More than twenty-five years ago, large numbers of parents of infants and toddlers made the switch from cloth diapers to disposable diapers. Disposable diapers offered harried parents the option of absorbing and containing their babies' biological wastes in a form that was easy to use (did not require the use of safety pins) and that was single use, thereby eliminating the need to launder cloth diapers. As part of the evolution of convenience associated with use of disposable diapers, cleaning of baby's skin during diaper changes switched from the use of wash clothes to the use of individual, premoistened sheets called "wet wipes". During the past decades, great research efforts have been made to improve the performance and fit of disposable diapers and the cleansing capabilities of wet wipes.

Disposable absorbent articles such as diapers, training pants, incontinence products and feminine care products are worn such that they are in direct contact with the skin of the wearer. An unavoidable consequence of the use of absorbent articles is that the skin is exposed more directly to various physical and biological insults. Consequently, the barrier function of the skin covered by the absorbent article is put at risk. In order to provide disposability, absorbent articles are primarily constructed of nonwoven materials. Even though nonwoven materials are engineered to have soft hand and drape, they rub against the skin and there is friction. Such friction constitutes one form of physical insult to the skin barrier. Friction against the skin barrier also occurs with the use of absorbent tissues and wet wipes. Absorbent tissue and wet wipe products are frequently used for cleansing the skin areas covered by absorbent articles. Absorbent tissue and wipe products are necessary for removing biological waste materials from the skin.

In addition to these physical insults, skin covered by absorbent articles is also frequently exposed to biological insults. Biological fluids, such as urine, feces, vaginal secretions and nasal secretions, may contain a variety of components that can damage the skin barrier. Examples of these components include proteases, lipases and bile acids. Once the skin barrier is compromised, these components, in addition to other constituents of biological fluids, can initiate or exacerbate inflammation of the skin.

Diaper dermatitis is a genre of skin conditions that, in large part, originate from impaired skin barrier function. Similar "dermatitis"-type conditions can occur with the use of other absorbent articles, too. Impairment of the skin barrier can result from a variety of factors, including: increased skin hydration due to the occlusion of the skin caused by diapers, enzymatic skin damage due to fecal and urinary enzymes, and physical damage caused by friction against the diaper surface and repeated cleaning of the skin with absorbent tissues or wet wipes.

Excessive hydration of the skin also has a negative effect on the skin barrier. The hydration level of diapered skin, for example, may reach between five to ten times that of undiapered skin. Frequent contact of diapered skin with urine may also contribute to increased skin hydration. Increased skin hydration disrupts skin lipid organization in the stratum corneum. This disruption may increase the permeability of the skin to irritants from feces and urine, thus increasing the risk of skin inflammation.

Disposable absorbent articles such as diapers, training pants, adult incontinence products, absorbent under pants, feminine care products and nursing pads have been used to absorb body fluids and leave the skin dry. Disposable absorbent articles of this type generally include a liquid impermeable backsheet member, an absorbent core or assembly, and a liquid permeable body facing or liner material. The body facing or liner material comes into contact with the wearer's skin. While the body facing material is made of a soft, compliant material, the material rubs against the skin during use and may not leave the skin completely dry and free of the bodily fluids, such as solid or semi-solid waste, the absorbent article is trying to absorb. During frequent insults of bodily fluids and frequent use of disposable absorbent articles, the skin can become irritated and appear red and be sore to the touch.

Creams, lotions or ointments can be used to provide an artificial hydrophobic barrier on the skin and to treat skin conditions such as diaper rash. Continuing the evolution of convenience, presently available disposable diapers frequently include a lotion or ointment that is applied to the bodyfacing surface of the diaper liner. Desirably, during use, the lotion transfers to the skin of the diaper wearer and enhances or provides barrier function of/to the skin. The lotions are frequently formulated to provide stability on the diaper liner during shipping and storage and to transfer to skin during use at body temperature. The lotions are also typically formulated to reduce migration of the lotion across the liner surface and migration of the lotion through the liner toward the absorbent core. Though many lotion formulations have been developed, many of the formulations have a petrolatum or other hydrophobic base and therefore, are non-aqueous.

Wet wipes are typically provided to consumers in a pre-moistened condition. This eliminates the need for the user or caregiver to have to moisten the wipe during the process of changing an absorbent article. The solutions used to pre-moisten wet wipes are composed largely of hydrophilic solvents such as water but can include other ingredients such as surfactants to aid cleansing and compounds to improve the wettability of the wet wipe sheet.

Even though they are consistently used together with great frequency, disposable diapers and other disposable absorbent articles are typically packaged and sold separately from wet wipes. Separate packaging is more conducive to purchasing both items in large quantities for use at home. However, many people have recognized the benefits of having smaller quantities of both diapers and wet wipes available for use when away from home. Playing on the theme of convenience, many versions of individual packages of a disposable diaper with one or more wet wipes have been described. For example, various configurations for attaching wet wipes to disposable diapers have been described. Diapers having special folds or additional components to form pockets for holding the wet wipes have been considered. The benefits of these configurations include better convenience for away-from-home situations in order to avoid the need to carry full packages of products (diapers and wet wipes) around.

Even though the concepts of using diapers and wipes together and of packaging diapers and wipes together have been explored, no consideration has been given to leveraging the chemistries associated with each medium to deliver benefits to the wearer's skin. Thus, what is needed is a topically effective composition delivered from a bodyside or bodyfacing material of an absorbent article that protects, maintains, recovers or otherwise benefits skin barrier function against physical damage and irritants in biological fluids that is used systematically with a composition delivered from a wet wipe to the skin in order to reduce irritation, and enhance skin barrier function more than just using the wet wipe or composition delivered from the absorbent article alone.

## Summary of the Invention

In response to the difficulties and problems discussed above, a system for improving the skin health of wearers of absorbent articles has been discovered. The system includes an absorbent article having a non-aqueous skin care composition on a bodyfacing surface that transfers to the skin together with a wet wipe having a hydrophilic skin care solution that also transfers to the skin. The system of the invention can be used in conjunction with absorbent articles such as diapers, incontinence garments, feminine care products, training pants and diaper pants. The purposes and advantages of the present invention will be set forth in and apparent from the description that follows, as well as will be learned by practice of the invention. Additional advantages of the invention will be realized and attained by the compositions and articles particularly pointed out in the written description and claims hereof, as well as from the appended drawings.

In one aspect, the present invention relates to a system for improving the skin health of a wearer of absorbent articles. The system includes a disposable absorbent article that has an outer cover, a bodyside liner, an absorbent body and a skin care composition. The bodyside liner is typically liquid permeable and defines a bodyfacing surface. The bodyside liner is connected in a generally superposed relation to the outer cover. The absorbent body is located between the bodyside liner and the outer cover. The skin care composition is on a portion or the entire bodyfacing surface of the bodyside liner. The skin care composition can be generally solid, semi-solid or liquid. The skin care composition may be in a variety of forms, including, but not limited to, emulsions, lotions, creams, ointments, salves, suspensions and gels. The skin care composition can be applied to the bodyside liner using a variety of techniques including foam application, spraying, slot coating and printing. The present invention also encompasses technology that would permit integration of the skin care composition directly with fibers or other materials used to form the bodyside liner. The skin care compositions can be applied to the bodyfacing surface in amounts of from about 0.1 grams per meter squared $(g/m^2)$ to about 30 $g/m^2$. The skin care compositions of the invention could also be applied to or be present on other skin contacting surfaces of absorbent articles such as the waist and leg elastics and the containment flaps.

The skin care compositions of the invention can include from about 40 to about 95 percent by weight of one or more emollients. Emollients are skin conditioning ingredients that help to soften, smooth, plasticize, lubricate, moisturize, improve the appearance of, improve the feel of and protect the skin. More specifically, the compositions include from about 50 to about 85 percent by weight of emollient(s). Even more specifically, the compositions include from about 60 to about 75 percent by weight of emollient(s). Suitable emollients include, but are not limited to, petroleum based oils, petrolatum, vegetable oils, hydrogenated vegetable oils, animal oils, hydrogenated animal oils, mineral oils, alkyl dimethicones, alkyl methicones, alkyldimethicone copolyols, phenyl silicones, alkyl trimethylsilanes, dimethicone, lanolin and its derivatives, esters, branched esters, glycerol esters and their derivatives, propylene glycol esters and their derivatives, alkoxylated carboxylic acids, alkoxylated acids, fatty alcohols, triglycerides, alkyl hydroxystearates and mixtures of such compounds.

In addition to the components already described, the skin care compositions of the invention may further include from about 5 to about 60 percent by weight of one or more compounds acting as viscosity enhancers that increase the meltpoint viscosity of the emollients of the skin care composition. More specifically, the skin care compositions include from about 15 to about 40 percent by weight of one or more viscosity enhancers. Even more specifically, the skin care compositions include from about 20 to about 35 percent by weight of viscosity enhancer(s). The viscosity enhancer increases the meltpoint viscosity of the skin care compositions to have a high viscosity under low shear and at the "hot box car" stability temperature of approximately 54.5°C. Having high viscosity (>50,000 centipoise) at elevated temperatures prevents the skin care compositions from migrating into or away from the materials to which they are applied. However, the viscosity enhancer component also provides a low viscosity (<5,000 centipoise) for the skin care compositions under high shear and at processing temperatures. The viscosity enhancers of the invention are capable of providing a desirable viscosity, depending on shear and temperature conditions, for skin care compositions having a range of melting points. While it is desirable for skin care compositions of the invention to have increased viscosity under "hot box car" stability conditions, the increased viscosity can be maintained, in part, through the use of one or more viscosity enhancers up to the melting point of the particular skin care composition. Typically, process temperatures are approximately 5°C above the melting point of the skin care composition.

Viscosity enhancers are typically selected to provide the desired viscosity and transfer properties for an emollient or mixture of emollients. For skin care compositions applied to the bodyfacing surfaces of absorbent articles, it is desirable to provide a relatively viscous skin care composition at room temperature to maintain stability on the bodyfacing surfaces. At body temperature, it is desirable for the skin care compositions to begin to soften in order to improve transfer to the skin of the wearer. When the skin care compositions are transferred to the skin, they improve the barrier function of the skin. The non-aqueous nature of the skin care compositions makes them suitable for delivering skin barrier enhancing excipients to the skin. Because the skin care compositions serve to generally protect and maintain barrier functions, it is desirable for the wearer's skin to have as much contact with the skin care compositions as possible. Maximum contact with the skin is achieved by delivering the skin care compositions from at least the bodyside liners of the absorbent articles of the systems of the invention.

Examples of suitable viscosity enhancers include, but are not limited to, polyolefin resins, lipophilic/oil thickeners, ethylene/vinyl acetate copolymers, natural clays, synthetic analogs of natural clays, organically modified clays, quaternary modified clays, quaternary starch compounds, polyethylene, silica, silica silylate, silica methyl silylate, colloidal silicone dioxide, alkyl hydroxy ethyl cellulose, other organically modified celluloses, PVP/decane copolymer, PVM/MA decadiene crosspolymer, PVP/eicosene copolymer, PVP/hexadecane copolymer, microcrystalline wax, hexadecyl-cosanyl-hexacosanate, shellac wax, glycol montanate, PEG-12 carnauba, synthetic paraffin, ozokerite, $C_{20}$-$C_{40}$ alkyl hydroxystearyl stearate, polyperfluoromethylisopropylether montan wax, magnesium aluminum silicate, polymethacrylate polymers, polystyrene copolymers and mixtures of these compounds.

The skin care compositions of the invention may also include from about 5 to about 55 percent by weight of one or more solidifying agents. More specifically, the skin care compositions include from about 15 to about 45 percent by weight of solidifying agents. Even more specifically, the skin care compositions include from about 25 to about 35 percent by weight of solidifying agents. A solidifying agent is a material capable of solidifying the skin care composition so that the skin care composition is solid at room temperature and has a penetration hardness of at least 5 mm. More specifically, the solidifying agent includes one or more materials that are capable of solidifying a natural fats/oils and emollient combination so as to have a penetration hardness of 5 to about 365 mm at 25°C. Further, the solidifying agent solidifies the emollient (or the fat/oil/emollient combination when fats and oils are used in the composition) so that it has a melting point between 32°C and 100°C. One or more solidifying agents can be selected from alkyl siloxanes (with a melting point greater than 35°C), polymers, waxes (animal, vegetable or mineral), synthetic waxes, hydrogenated vegetable/animal oils having a melting point of 35°C or greater, fatty acid esters and branched esters having a melting point of 35°C or greater, alkyl hydroxystearates (>$C_{16}$), alkoxylated alcohols and alkoxylated carboxylic acid.

Examples of suitable solidifying agents include, but are not limited to, the following compounds: beeswax, behenyl behenate, behenyl benzoate, branched esters, candelilla wax, carnauba wax, synthetic carnauba wax, PEG-12 carnauba wax, cerasin, microcrystalline wax, hydrogenated microcrystalline wax, hexadecylcosanyl hexacosanate, polyperfluoromethylisopropylether montan wax, alkylmethylsiloxanes, glycol montanate, jojoba wax, lanolin wax, ozokerite, paraffin, synthetic paraffin, polyethylene, $C_{20}$-$C_{40}$ alkyl hydroxystearyl stearate, $C_{30}$ alkyl dimethicone, cetyl esters, zinc stearate, shellac wax, hydrogenated cottonseed oil, hydrogenated squalene, hydrogenated jojoba oil and mixtures thereof.

The skin care compositions of the invention can also include from about 0.1 to about 55 percent by weight of natural fats or natural oils that contain essential and non-essential fatty acids. More specifically, the skin care compositions can include from about 5 to about 40 percent by weight of natural fats or natural oils. Desirably, the skin care compositions of the invention include from about 15 to about 30 percent by weight of natural fats, natural oils or mixtures of both. Natural fats and oils include fats, oils, essential oils, fatty acids, fatty alcohols, phospholipids and mixtures of these compounds. The natural fats and oils can be similar to the lipids that are present in healthy skin in order to mimic the naturally present lipids. Synthetic or synthetically modified fats and oils could potentially also be used if they functioned in the same manner as their natural counterparts. Examples of fats and oils include, but are not limited to, Avocado Oil, Apricot Oil, Babassu Oil, Borage Oil, Camellia Oil, Canola Oil, Castor Oil, Coconut Oil, Corn Oil, Cottonseed Oil, Evening Primrose Oil, Hydrogenated Cottonseed Oil, Hydrogenated Palm Kernel Oil, Maleated Soybean Oil, Meadowfoam Oil, Palm Kernel Oil, Peanut Oil, Rapeseed Oil, Safflower Oil, Sphingolipids, Sweet Almond Oil, Tall Oil, Lanolin, Lanolin Alcohol, Lauric Acid, Palmitic Acid, Stearic Acid, Linoleic Acid, Stearyl Alcohol, Lauryl Alcohol, Myristyl Alcohol, Behenyl Alcohol, Rose Hip Oil, Calendula Oil, Chamomile Oil, Eucalyptus Oil, Juniper Oil, Sandlewood Oil, Tea Tree Oil, Sunflower Oil, Soybean Oil and mixtures thereof.

The skin care compositions can also include sterols, sterol derivatives or mixtures of both in an amount of from about 0.1 to about 10 percent by weight. Sterols and sterol derivatives include compounds such as ß-sterols with a tail on the 17 position and no polar groups, such as cholesterol, $C_{10}$-$C_{30}$ cholesterol/lanosterol esters, tall oil sterols, soy sterols, sterol esters and mixtures of these compounds. More specifically, the skin care compositions include from about 0.5 to about 5 percent by weight of sterols, sterol derivatives or mixtures of both. Even more specifically, the skin care compositions include from about 0.8 to about 1 percent by weight of the sterol compounds. Examples of suitable sterol compounds include, but are not limited to, cholesterol, sitosterol, stigmasterol, and ergosterol, as well as, $C_{10}$-$C_{30}$ cholesterol/lanosterol esters, cholecalciferol, cholesteryl hydroxystearate, cholesteryl isostearate, cholesteryl stearate, 7-dehydrocholesterol, dihydrocholesterol, dihydrocholesteryl octyldecanoate, dihydrolanosterol, dihydrolanosteryl octyldecanoate, ergocalciferol, tall oil sterol, soy sterol acetate, lanasterol, soy sterol, avocado sterols, "AVOCADIN" (available from Croda Ltd. of Parsippany, New Jersey), sterol esters and mixtures thereof.

The system of the invention also includes a wet wipe that works synergistically with the absorbent article to improve the skin health of wearers of absorbent articles. The wet wipe includes a nonwoven substrate and a skin care solution. The wet wipes of the present invention comprise a single layer or a layered basesheet that contains a liquid. The liquid is a skin care solution that can be absorbed into the wet wipe basesheet and may include any components customary to wet wipes in order to provide desirable wiping properties. Typically, the components have included water, emollients, surfactants, fragrances, preservatives, chelating agents, pH buffers or combinations thereof as are well known to those skilled in the art. The skin care solutions of the invention can include hydrophilic solvent, surfactant and extracted botanical actives.

The skin care solutions of the invention can include from about 70 to about 99 percent by weight of one or more hydrophilic solvents. Desirably, the hydrophilic solvent is water. The water contained in the solution may be a mixture of water and alcohol. The preferred alcohols are ethanol and isopropyl alcohol. The amount of alcohol in the water is up to about 70 weight percent of the water and alcohol solution. More preferably, the amount of alcohol in the water is from about 40 to about 60 weight percent of the water and alcohol solution.

The skin care solutions of the invention can also include from about 0 to about 30 percent by weight of one or more surfactants. More specifically, the skin care solution can include from about 0.5 to about 20 percent by weight of surfactant. Desirably, the skin care solution can include from about 1 to about 15 percent by weight of surfactant. The surfactants of the skin care solution can include anionic, nonionic, cationic, zwitterionic, amphoteric and polymeric surfactants and mixtures thereof. One who is skilled in the art will recognize the functions and use of each of these classes of surfactants. Some botanicals and other skin care health benefiting agents are lipophilic and would require emulsification into the wet wipe solution. In order to emulsify lipophilic ingredients it may be necessary to use surfactants having an HLB of 6 to about 18 to stablize these lipophilic ingredient in the wet wipe solution.

Desirably, the surfactant of the skin care solution is selected from emulsifying wax NF, glyceryl stearate, glyceryl stearate SE glycol stearate, glycol stearate SE, glycereth-20 Stearate, glyceryl behenate, glyceryl hydroxystearate, glyceryl laurate SE, glyceryl oleate, glyceryl oleate SE, propylene glycol oleate, propylene glycol oleate SE, propylene glycol stearate, propylene glycol stearate SE, sorbitan stearate, sorbitan trioleate acrylates/$C_{10-30}$ alkyl acrylate cross-polymer and mixtures thereof.

The compositions of the invention can also include from about 0.1 to about 10 percent by weight of one or more extracted botanical actives. More specifically, the compositions can include from about 0.5 to about 8 percent by weight of one or more extracted botanical actives. Even more specifically, the compositions include from about 1 to about 5 percent by weight of extracted botanical actives. Extracted botanical actives can include any water-soluble or oil-soluble active extracted from a particular plant. In addition, the botanical extracted actives can be supplied as a powder. The botanical actives are actives extracted from echinacea, yucca, willow herb, green tea, black tea, oolong tea, Chinese tea, constituents of tea extract and mixtures thereof. Echinacea actives may be obtained from the following echinacea species: *Echinacea angustifolia, Echinacea purpurea,* and *Echinacea pallida.* Varieties of black tea include Flowery Orange Pekoe, Golden Flowery Orange Pekoe and Fine Tippy Golden Flowery Orange Pekoe. Varieties of green tea include Japanese and Green Darjeeling.

Botanicals are primarily extracts of the plants from which they originate and botanicals are available from suppliers as part of a composition that may also contain an extracting solvent. Amounts of the botanicals in the compositions of the invention in terms of active component (not extract) may range from about 0.000001 to about 10% by weight. Desirably, the amount of active botanical is from about 0.00001 to about 5% and more desirably from about 0.0001 to about 1% by weight of the composition. Further, it is also desirable that the amount of active botanical is from about 0.0001 to about 0.5% of the composition and more desirably from about 0.001 to about 0.1% by weight of the composition.

The skin care solution incorporated into the wet wipe of the skin health improving system of the invention can include components in addition to the hydrophilic solvent, surfactant and extracted botanical active components. For example, the skin care solution can include an oil in water emulsion formed by incorporation of from about 0.1 to about 30 percent by weight of natural fats or oils with the hydrophilic solvent. More specifically, from about 0.5 to about 10 percent by weight of natural fats or oils can be used to transform the skin care solution into an emulsion. Even more specifically, from about 1 to about 5 percent by weight of natural fats or oils can be used to form an emulsion with the hydrophilic solvent of the skin care solution. Suitable natural fats or oils can be selected from avocado oil, apricot oil, babassu oil, borage oil, camellia oil, canola oil, castor oil, coconut oil, corn oil, cottonseed oil, evening primrose oil, hydrogenated cottonseed oil, hydrogenated palm kernel oil, maleated soybean oil, meadowfoam oil, palm kernel oil, phospholipids, rapeseed oil, palmitic acid, stearic acid, linoleic acid, stearyl alcohol, lauryl alcohol, myristyl alcohol, benenyl alcohol, rose hip oil, sunflower oil, soybean oil and mixtures of such natural fats and oils.

The skin care solutions of the invention can also incorporate sterols and sterol derivatives in such a way that an emulsion is formed with the hydrophilic solvent. For example, the skin care solution can include from about 0.1 to about 10 percent by weight of one or more sterols or sterol derivatives. More specifically, the skin care solution can include from about 0.5 to about 5 percent by weight of sterols or sterol derivatives. Even more specifically, the skin care solution can include from about 0.8 to about 3 percent by weight of sterols or sterol derivatives where the sterols and sterol derivatives form an emulsion with the hydrophilic solvent and other components. Suitable sterols and sterol derivatives can be selected from cholesterol, sitosterol, stigmasterol, ergosterol, lanasterol, soy sterol, avocado sterols, cholesterol esters, sterol esters, lanolin and mixtures thereof.

The skin care solutions of the invention can also include a humectant component. Humectants can be included in order to increase the water content of the top layers of the skin. Humectant materials include primarily hydroscopic ingredients. The skin care solutions of the invention can include from about 0.1 to about 30 percent by weight of one or more humectants. More specifically, the skin care solutions can include from about 0.5 to about 20 percent by weight of one or more humectants. Even more specifically, the skin care solutions can include from about 1 to about 10 percent by weight of one or more humectants. Suitable humectants may be selected from acetamide MEA, aloe vera gel, arginine PCA, chitosan PCA, copper PCA, corn glycerides, dimethyl imidazolidinone, fructose, glucamine, glucose, glucose glutamate, glucuronic acid, glutamic acid, glycereth-7, glycereth-12, glycereth-20, glycereth-26, glycerin, honey, hydrogenated honey, hydrogenated starch hydrolysate, hydrolyzed corn starch, lactamide MEA, lactic acid, lactose lysine PCA, mannitol, methyl gluceth-10, methyl gluceth-20, PCA, PEG-2 lactamide, PEG-10 propylene glycol, polyamino sugar condensate, potassium PCA, propylene glycol, propylene glycol citrate, saccharide hydrolysate, saccharide isomerate, sodium aspartate, sodium lactate, sodium PCA, sorbitol, TEA-lactate, TEA-PCA, urea, xylitol and mixtures of such compounds.

The systems of the invention contemplate that absorbent articles including the described skin care compositions and wet wipes including the described skin care solutions will be used together as such articles and wipes are typically used together to provide containment of bodily exudates and cleansing of the skin areas exposed to the bodily exudates. In order to obtain the benefits of the invention, it is not necessary for the wet wipe component of the system to be used with every change of the absorbent article component. The skin of the wearer of the absorbent article is exposed to the skin care composition during the time that the absorbent article is worn. With the systems of the invention, the wet wipe component can be used to cleanse the skin of the absorbent article wearer before the absorbent article component is donned. The wet wipe component can also be used to cleanse the skin of the absorbent article wearer when the absorbent article is removed from the wearer. More than one wet wipe can be used with each change of absorbent article.

The benefits of the invention can be derived from even a single use of the absorbent article + wet wipe system of the invention. The predicted improvements in skin health would be expected to increase with more frequent usage of the absorbent article + wet wipe system. The skin care composition applied to the absorbent articles is believed to deliver a protective and enhanced barrier effect to the skin. The skin care solution applied to the wet wipes is believed to deliver an anti-irritant effect to the skin. The combination of the absorbent article and the wet wipe can be used at least one time a day. Further, the combination of the absorbent article and the wet wipe can be used more than once a day; the combination could be used two times, three times or however many times the absorbent article is changed throughout the day. It is also possible that the skin care composition and the skin care solution could be selected to deliver sufficient efficacy in a single usage of the combination that the desired improvement in skin health is achieved.

The systems of the invention are configured to deliver skin health benefits best delivered through a non-aqueous vehicle from the bodyfacing surfaces of absorbent articles and to deliver skin health benefits best delivered through a hydrophilic vehicle from a wet wipe.

In furtherance of the achievement of improving the skin health of wearers of absorbent articles, the skin care compositions and skin care solutions of the invention may also include active ingredients such as those ingredients that may be useful for treating skin irritations such as diaper rash. Examples of such active ingredients include allantoin and its derivatives, aloe, aluminum hydroxide gel, calamine, cocoa butter, cod liver oil, dimethicone, glycerin, kaolin and its derivatives, lanolin and its derivatives, mineral oil, petrolatum, shark liver oil, talc, topical starch, zinc acetate, zinc carbonate, zinc oxide and mixtures of these ingredients. Some of the ingredients listed as possible active ingredients for treating the skin can also be used as emollients.

Ranges are used to describe the relative quantities of components in the compositions and solutions of the invention and ranges are used to describe the relative physical properties of the compositions and solutions of the invention. It is understood that the ranges are by way of illustration only and that one of skill in the art would recognize that the nature of the specific compositions dictates the levels to be applied to achieve the desired results. The levels of components are ascertainable by routine experimentation in view of the present disclosure.

Usage of the systems of the invention advantageously protect the skin barrier and subdue inflammation in such a way not observed with conventional absorbent articles and wet wipes. It is to be understood that both the foregoing general description and the following detailed description are exemplary and are intended to provide further explanation of the invention claimed. The accompanying drawings, that are incorporated in and constitute part of this specification, are included to illustrate and provide a further understanding of the systems of the invention. Together with the description, the drawings serve to explain the various aspects of the invention.

## Brief Description of the Drawings

The present invention will be more fully understood and further advantages will become apparent when reference is made to the following detailed description of the invention and the accompanying drawings. The drawings are merely representative and are not intended to limit the scope of the claims. Like parts of the absorbent articles depicted in the drawings are referred to by the same reference numerals.

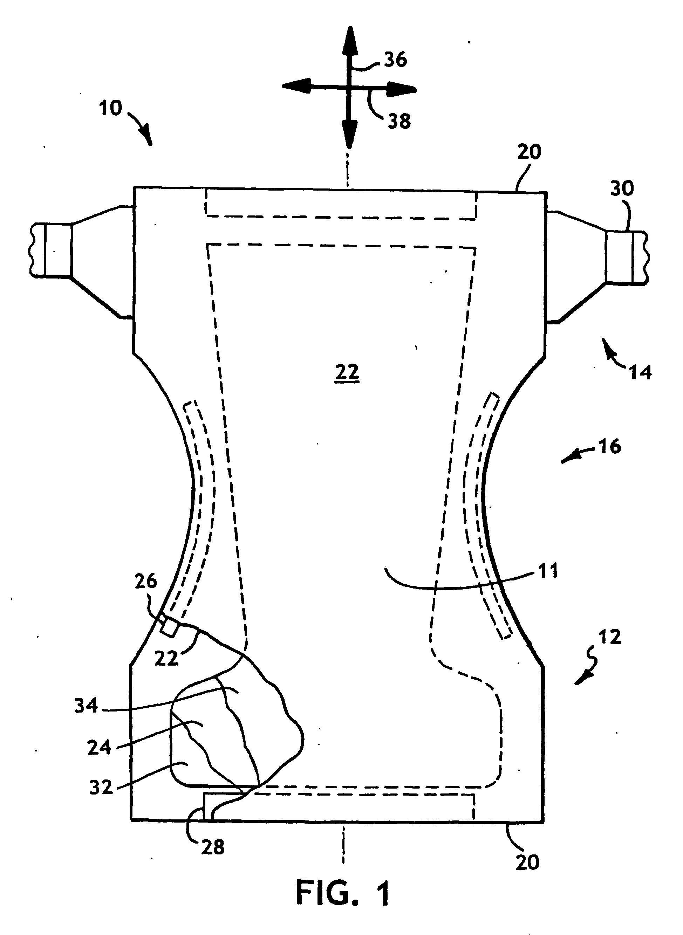

Fig. 1 representatively shows a partially cut away, top plan view of an absorbent article according to one aspect of the invention in a stretched and laid flat condition with the surface of the article that contacts the skin of the wearer facing the viewer.

## Detailed Description of the Invention

The present invention is directed to delivering both non-aqueous and hydrophilic compositions to the skin of persons wearing disposable absorbent articles. The non-aqueous and hydrophilic compositions work together to provide benefits not achievable through their use by themselves. More specifically, the present invention is directed to a system for improving skin health in which a non-aqueous skin care composition is delivered from an absorbent article and a hydrophilic skin care solution is delivered from a wet wipe.

The present disclosure of the invention will be expressed in terms of its various components, elements, constructions, configurations, arrangements and other features that may also be individually or collectively referenced by the term, "aspect(s)" of the invention, or other similar terms. It is contemplated that the various forms of the disclosed invention may incorporate one or more of its various features and aspects, and that such features and aspects may be employed in any desired, operative combination thereof.

It should also be noted that when employed in the present disclosure, the terms "comprises", "comprising" and other derivatives from the root term "comprise" are intended to be open-ended terms that specify the presence of any stated features, elements, integers, steps, or components, and are not intended to preclude the presence or addition of one or more other features, elements, integers, steps, components or groups thereof.

The present invention encompasses compositions as they are applied to the bodyfacing materials of absorbent articles and to wet wipes and absorbent articles and wet wipes including compositions. The following detailed description will be made in the context of one type of absorbent article, a disposable diaper that is adapted to be worn by infants about their lower torso. It is readily apparent, however, that the system of the present invention would also be suitable for use with other types of absorbent articles, such as feminine care pads, incontinence garments, training pants and prefastened or refastenable diaper pants. The description of the systems of the invention will begin with a general description of a disposable diaper type of absorbent article. The next portion of the detailed description will be followed by a description of the skin care compositions that can be applied to the disposable diaper or other absorbent article. Following the descriptions of the absorbent articles and skin care compositions will be descriptions of wet wipes and the skin care solutions applied to the wet wipes.

Fig. 1 is a representative plan view of a disposable diaper 10 of the present invention in a flat-out, uncontracted state (i.e., with all elastic induced gathering and contraction removed). The bodyfacing surface 11 of the diaper 10, that is, the surface 11 of the diaper 10 that contacts the wearer is facing the viewer. The compositions of the invention can be applied to one or more bodyfacing materials that are components of the diaper 10. As used herein, the term 'bodyfacing material' includes, but is not limited to, materials such as the bodyside liner or topsheet, elastic material, tissue, intake and distribution material, absorbent material, and backsheet material. Each of these materials and components of a diaper 10 are described more fully herein. The compositions of the invention are applied to one or more of the bodyfacing materials in order to have maximum exposure and opportunity to transfer to the skin of the wearer of the diaper. The bodyfacing material of the present invention can be a single layer or multi-layered.

With reference to Fig. 1, the diaper 10 generally defines a front waist section 12, a rear waist section 14, and an intermediate section 16 that interconnects the front and rear waist sections 12 and 14. The front and rear waist sections 12 and 14 include the general portions of the diaper 10 that are constructed to extend substantially over the wearer's front and rear abdominal regions, respectively, during use. The intermediate section 16 of the diaper 10 includes the general portion of the diaper 10 that is constructed to extend through the wearer's crotch region between the legs.

The diaper 10 includes a vapor permeable backsheet or outer cover 20, a liquid permeable topsheet or bodyside liner 22 positioned in facing relation with the outer cover 20, and an absorbent body 24, such as an absorbent pad, which is located between the outer cover 20 and the bodyside liner 22. The outer cover 20 defines a length and a width that, in the illustrated aspect, coincide with the length and width of the diaper 10. The absorbent body 24 generally defines a length and width that are less than the length and width of the outer cover 20, respectively. Thus, marginal portions of the diaper 10, such as marginal sections of the outer cover 20, may extend past the terminal edges of the absorbent body 24. In the illustrated aspects, for example, the outer cover 20 extends outwardly beyond the terminal marginal edges of the absorbent body 24 to form side margins and end margins of the diaper 10. The bodyside liner 22 is generally coextensive with the outer cover 20 but may optionally cover an area that is larger or smaller than the area of the outer cover 20, as desired. In other words, the bodyside liner 22 is connected in superposed relation to the outer cover 20. The outer cover 20 and bodyside liner 22 are intended to face the garment and body of the wearer, respectively, while in use.

To provide improved fit and to help reduce leakage of body exudates from the diaper 10, the diaper side margins and end margins may be elasticized with suitable elastic members, such as single or multiple strands of elastic. The elastic strands may be composed of natural or synthetic rubber and may optionally be heat shrinkable or heat elasticizable. For example, as representatively illustrated in Fig. 1, the diaper 10 may include leg elastics 26 which are constructed to operably gather and shirr the side margins of the diaper 10 to provide elasticized leg bands which can closely fit around the legs of the wearer to reduce leakage and provide improved comfort and appearance. Similarly, waist elastics 28 can be employed to elasticize the end margins of the diaper 10 to provide elasticized waists. The waist elastics 28 are configured to operably gather and shirr the waist sections to provide a resilient, comfortably close fit around the waist of the wearer. In the illustrated aspects, the elastic members are illustrated in their uncontracted, stretched condition for the purpose of clarity.

Fastening means, such as hook and loop fasteners 30, are employed to secure the diaper 10 on a wearer. Alternatively, other fastening means, such as buttons, pins, snaps, adhesive tape fasteners, cohesives, mushroom-and-loop fasteners, or the like, may be employed. Additionally, more than two fasteners can be provided, particularly if the diaper 10 is to be provided in a prefastened configuration. The fasteners can vary in size and form.

The diaper 10 may further include other layers between the absorbent body 24 and the bodyside liner 22 or outer cover 20. For example, as representatively illustrated in Figs. 1 and 2, the diaper 10 may include a ventilation layer 32 located between the absorbent body 24 and the outer cover 20 to insulate the outer cover 20 from the absorbent body 24, to improve air circulation and to effectively reduce the dampness of the garment facing surface of the outer cover 20. The ventilation layer 32 may also assist in distributing fluid exudates to portions of the absorbent body 24 that do not directly receive the insult. The diaper 10 may also include a surge management layer 34 located between the bodyside liner 22 and the absorbent body 24 to prevent pooling of the fluid exudates and further improve air exchange and distribution of the fluid exudates within the diaper 10.

The diaper 10 may be of various suitable shapes. For example, the diaper may have an overall rectangular shape, T-shape or an approximately hourglass shape. In the shown aspect, the diaper 10 has a generally I-shape. The diaper 10 further defines a longitudinal direction 36 and a lateral direction 38. Other suitable diaper components that may be incorporated on absorbent articles of the present invention include containment flaps, waist flaps, elastomeric side panels, and the like which are generally known to those skilled in the art. Likewise, if the diaper 10 is to be sold in a prefastened condition, the diaper 10 may have passive bonds (not shown) that join the rear waist section 14 with the front waist section 12.

Examples of diaper configurations suitable for use in connection with the instant application that may include other diaper components suitable for use on diapers are described in U.S. Patent 4,798,603 issued January 17, 1989, to Meyer et al.; U.S. Patent 5,176,668 issued January 5, 1993, to Bernardin; U.S. Patent 5,176,672 issued January 5, 1993, to Bruemmer et al.; U.S. Patent 5,192,606 issued March 9, 1993, to Proxmire et al.; U.S. Patent 5,496,298 issued March 5, 1996 to Kuepper et al.; U.S. Patent 5,509,915 issued April 23, 1996 to Hanson et al.

The various components of the diaper 10 are integrally assembled together employing various types of suitable attachment means, such as adhesive, sonic bonds, thermal bonds or combinations thereof. In the shown aspect, for example, the bodyside liner 22 and outer cover 20 are assembled to each other and to the absorbent body 24 with lines of adhesive, such as a hot melt, pressure-sensitive adhesive. Similarly, other diaper components, such as the elastic members 26 and 28, fastening members 30, and ventilation and surge layers 32 and 34 may be assembled into the diaper 10 by employing the above-identified attachment mechanisms.

The outer cover 20 of the diaper 10, as representatively illustrated in Fig. 1, is composed of a substantially vapor permeable material. The permeability of the outer cover 20 is configured to enhance the breathability of the diaper 10 and to reduce the hydration of the wearer's skin during use without allowing excessive condensation of vapor, such as urine, on the garment facing surface of the outer cover 20 that can undesirably dampen the wearer's clothes. The outer cover 20 is generally constructed to be permeable to at least water vapor and has a water vapor transmission rate of at least about 1000 $g/m^2/24$ hr., desirably at least about 1500 $g/m^2/24$ hr, more desirably at least about 2000 $g/m^2/24$ hr., and even more desirably at least about 3000 $g/m^2/24$ hr. For example, the outer cover 20 may define a water vapor transmission rate of from about 1000 to about 6000 $g/m^2/24$ hr. Materials that have a water vapor transmission rate less than those above do not allow a sufficient amount of air exchange and undesirably result in increased levels of skin hydration.

The outer cover 20 is also desirably substantially liquid impermeable. For example, the outer cover 20 may be constructed to provide a hydrohead value of at least about 60 cm, desirably at least about 80 cm, and more desirably at least about 100 cm when subjected to the Hydrostatic Pressure Test. Materials that have hydrohead values less than those above undesirably result in the strike through of liquids, such as urine, during use. Such fluid strike through can undesirably result in a damp, clammy feeling on the outer cover 20 during use. The methods by which Water Vapor Transmission Rate and Hydrostatic Pressure can be measured are described in U.S. Patent 6,217,890 issued April 17, 2001 to Paul et al.

The outer cover 20 may be composed of any suitable materials that either directly provide the above desired levels of liquid impermeability and air permeability or, in the alternative, materials that can be modified or treated in some manner to provide such levels. In one aspect, the outer cover 20 may be a nonwoven fibrous web constructed to provide the required level of liquid impermeability. For example, a nonwoven web composed of spunbond or meltblown polymer fibers may be selectively treated with a water repellent coating or laminated with a liquid impermeable, vapor permeable polymer film to provide the outer cover 20. In a particular aspect of the invention, the outer cover 20 may include a nonwoven web composed of a plurality of randomly deposited hydrophobic thermoplastic meltblown fibers that are sufficiently bonded or otherwise connected to one another to provide a substantially vapor permeable and substantially liquid impermeable web. The outer cover 20 may also include a vapor permeable nonwoven layer that has been partially coated or otherwise configured to provide liquid impermeability in selected areas.

Examples of suitable materials for the outer cover 20 are also described in U.S. Patent No. 5,482,765 issued January 9, 1996 in the name of Bradley et al. and entitled "NONWOVEN FABRIC LAMINATE WITH ENHANCED BARRIER PROPERTIES"; U.S. Patent No. 5,879,341 issued March 9, 1999 in the name of Odorzynski et al. and entitled "ABSORBENT ARTICLE HAVING A BREATHABILITY GRADIENT"; U.S. Patent No. 5,843,056 issued December 1, 1998, in the name of Good et al. and entitled "ABSORBENT ARTICLE HAVING A COMPOSITE BREATHABLE BACKSHEET"; and U.S. Patent No. 6,309,736 issued October 30, 2001, in the name of McCormack et al. and entitled "LOW GAUGE FILMS AND FILM/NONWOVEN LAMINATES".

In a particular aspect, the outer cover 20 is provided by a microporous film/nonwoven laminate material that includes a spunbond nonwoven material laminated to a microporous film. The spunbond nonwoven comprises filaments of about 2 dtex (1.8 denier) extruded from a copolymer of ethylene with about 3.5 weight percent propylene and defines a basis weight of from about 17 to about 25 grams per square meter. The film includes a cast coextruded film having calcium carbonate particles therein and defines a basis weight of about 58 grams per square meter prior to stretching. The film is preheated, stretched and annealed to form the micropores and then laminated to the spunbond nonwoven. The resulting microporous film/nonwoven laminate based material has a basis weight of from about 30 to about 60 grams per square meter and a water vapor transmission rate of from about 3000 to about 6000 $g/m^2/24$ hr. Examples of such film/nonwoven laminate materials are described in more detail in U.S. Patent 6,309,736 issued October 30, 2001, in the name of McCormack et al.

In another aspect, the outer cover 20 is provided by an extensible material. Further, the outer cover 20 can also be provided by a material having stretch in both the longitudinal 36 and lateral 38 directions. Extensible and stretchable outer cover materials can be used in absorbent articles to provide various benefits including better fitting articles.

The bodyside liner 22, as representatively illustrated in Fig. 1, defines a bodyfacing surface 11 that is compliant, soft feeling, and nonirritating to the wearer's skin. Further, the bodyside liner 22 may be less hydrophilic than the absorbent body 24, to present a relatively dry surface to the wearer, and may be sufficiently porous to be liquid permeable, permitting liquid to readily penetrate through its thickness. A suitable bodyside liner 22 may be manufactured from a wide selection of web materials, such as porous foams, reticulated foams, apertured plastic films, natural fibers (for example, wood or cotton fibers), synthetic fibers (for example, polyester or polypropylene fibers), or a combination of natural and synthetic fibers. The bodyside liner 22 is suitably employed to help isolate the wearer's skin from liquids held in the absorbent body 24.

Various woven and nonwoven fabrics can be used for the bodyside liner 22. For example, the bodyside liner 22 may be composed of a meltblown or spunbond web of polyolefin fibers. The bodyside liner 22 may also be a bonded-carded web composed of natural and/or synthetic fibers. The bodyside liner 22 may be composed of a substantially hydrophobic material, and the hydrophobic material may, optionally, be treated with a surfactant or otherwise processed to impart a desired level of wettability and hydrophilicity. In a particular aspect of the present invention, the bodyside liner 22 includes a nonwoven, spunbond, polypropylene fabric composed of about 3.11-3.56 dtex (2.8-3.2 denier) fibers formed into a web having a basis weight of about 22 grams per square meter and a density of about 0.06 gram per cubic centimeter.

In a particular aspect of the present invention, the bodyside liner 22 may be surface treated with about 0.3 weight percent of a surfactant mixture that contains a mixture of AHCOVEL Base N-62 and GLUCOPON 220UP surfactants in about a 3:1 ratio based on a total weight of the surfactant mixture. The AHCOVEL Base N-62 surfactant is purchased from Hodgson Textile Chemicals Inc., a business having offices in Mount Holly, North Carolina, and includes a blend of hydrogenated ethoxylated castor oil and sorbitan monooleate in a 55:45 weight ratio. The GLUCOPON 220UP surfactant is purchased from Henkel Corporation and includes alkyl polyglycoside. The surfactant may also include additional ingredients such as aloe. The surfactant may be applied by any conventional means, such as spraying, printing, brush coating, foam or the like. The surfactant may be applied to the entire bodyside liner 22 or may be selectively applied to particular sections of the bodyside liner 22, such as the medial section along the longitudinal centerline of the diaper, to provide greater wettability of such sections.

The absorbent body 24 of the diaper 10, as representatively illustrated in Fig. 1, may suitably comprise a matrix of hydrophilic fibers, such as a web of cellulosic fluff, mixed with particles of a high-absorbency material commonly known as superabsorbent material. In a particular aspect, the absorbent body 24 includes a matrix of cellulosic fluff, such as wood pulp fluff, and superabsorbent hydrogel-forming particles. The wood pulp fluff may be exchanged with synthetic, polymeric, meltblown fibers or with a combination of meltblown fibers and natural fibers. The superabsorbent particles may be substantially homogeneously mixed with the hydrophilic fibers or may be nonuniformly mixed. Alternatively, the absorbent body 24 may include a laminate of fibrous webs and superabsorbent material or other suitable matrix for maintaining a superabsorbent material in a localized area.

The absorbent body 24 may have any of a number of shapes. For example, the absorbent body 24 may be rectangular, I-shaped, or T-shaped. It is generally preferred that the absorbent body 24 is narrower in the intermediate section than in the front or rear waist sections of the diaper 10. The absorbent body 24 may be provided by a single layer or, in the alternative, may be provided by multiple layers, all of which need not extend the entire length and width of the absorbent body 24. In a particular aspect of the invention, the absorbent body 24 can be generally T-shaped with the laterally extending cross-bar of the "T" generally corresponding to the front waist section 12 of the absorbent article for improved performance, especially for male infants. In the illustrated aspects, for example, the absorbent body 24 across the front waist section 12 of the article has a cross-directional width of about 18 centimeters, the narrowest portion of the intermediate section 16 has a width of about 7.5 centimeters and in the rear waist section 14 has a width of about 11.4 centimeters.

The size and the absorbent capacity of absorbent body 24 should be compatible with the size of the intended wearer and the liquid loading imparted by the intended use of the absorbent article. Further, the size and the absorbent capacity of the absorbent body 24 can be varied to accommodate wearers ranging from infants through adults. In addition, it has been found that with the present invention, the densities and/or basis weights of the absorbent body 24 can be varied. In a particular aspect of the invention, the absorbent body 24 has an absorbent capacity of at least about 300 grams of synthetic urine.

In aspects wherein the absorbent body 24 includes the combination of hydrophilic fibers and high-absorbency particles, the hydrophilic fibers and high-absorbency particles can form an average basis weight for the absorbent body 24 that is within the range of about 400-900 grams per square meter. In certain aspects of the invention, the average composite basis weight of such an absorbent body 24 is within the range of about 500-800 grams per square meter, and preferably is within the range of about 550-750 grams per square meter to provide the desired performance.

To provide the desired thinness dimension to the various configurations of the absorbent article of the invention, the absorbent body 24 can be configured with a bulk thickness that is not more than about 0.6 centimeters. Preferably, the bulk thickness is not more than about 0.53 centimeters, and more preferably is not more than about 0.5 centimeters to provide improved benefits. The bulk thickness is determined under a restraining pressure of 0.2 psi (1.38 kPa).

The high-absorbency material can be selected from natural, synthetic, and modified natural polymers and materials. The high-absorbency materials can be inorganic materials, such as silica gels, or organic compounds, such as crosslinked polymers. The term "crosslinked" refers to methods for effectively rendering normally water-soluble materials substantially water insoluble but swellable. Such methods include, for example, physical entanglement, crystalline domains, covalent bonds, ionic complexes and associations, hydrophilic associations such as hydrogen bonding, and hydrophobic associations or Van der Waals forces.

Examples of synthetic, polymeric, high-absorbency materials include the alkali metal and ammonium salts of poly(acrylic acid) and poly(methacrylic acid), poly(acrylamides), poly(vinyl ethers), maleic anhydride copolymers with vinyl ethers and alpha-olefins, poly(vinyl pyrrolidone), poly(vinyl morpholinone), poly(vinyl alcohol), and mixtures and copolymers thereof. Further polymers suitable for use in the absorbent body 24 include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthan gum, locust bean gum, and the like. Mixtures of natural and wholly or partially synthetic absorbent polymers can also be useful in the present invention.

The high absorbency material may be in any of a wide variety of geometric forms. As a general rule, it is preferred that the high absorbency material be in the form of discrete particles. However, the high absorbency material may also be in the form of fibers, flakes, rods, spheres, needles, or other forms that would serve the same purpose. In general, the high absorbency material is present in the absorbent body 24 in an amount of from about 5 to about 90 percent by weight, desirably in an amount of at least about 30 percent by weight, and even more desirably in an amount of at least about 50 percent by weight based on a total weight of the absorbent body 24. For example, in a particular aspect, the absorbent body 24 may include a laminate which includes at least about 50 percent by weight and desirably at least about 70 percent by weight of high-absorbency material overwrapped by a fibrous web or other suitable material for maintaining the high-absorbency material in a localized area.

An example of high-absorbency material suitable for use in the present invention is SANWET IM 3900 polymer available from Hoechst Celanese, a business having offices in Portsmouth, Virginia. Other suitable superabsorbents may include FAVOR SXM 880 polymer obtained from Stockhausen, a business having offices in Greensboro, North Carolina.

Optionally, a substantially hydrophilic tissue wrapsheet (not illustrated) may be employed to help maintain the integrity of the structure of the absorbent body 24. The tissue wrapsheet is typically placed about the absorbent body 24 over at least the two major facing surfaces thereof. The tissue wrapsheet can be composed of an absorbent cellulosic material, such as creped wadding or a high wet-strength tissue. In one aspect of the invention, the tissue wrapsheet can be configured to provide a wicking layer that helps to rapidly distribute liquid over the mass of absorbent fibers constituting the absorbent body 24.

The absorbent body 24 of the different aspects of the present invention further includes a plurality of zones of high air permeability which allow air and vapors to readily pass through the absorbent body 24 and through the vapor permeable outer cover 20 out of the diaper 10 into ambient air. For example, the absorbent body 24 may include a plurality of air passageways that provide the absorbent body 24 with zones or regions of high air permeability. The portions of the absorbent body 24 adjacent the air passageways provide zones or regions of high absorption. The zones of high air permeability are designed to provide the maximum air exchange from the absorbent body 24 while the zones of high absorption are designed to receive and hold the majority of the body exudates. The absorbent body 24 may define any number of zones of high air permeability that provide the improved air exchange. Desirably, the absorbent body 24 defines at least 3 and more desirably at least 5 different zones of high air permeability for improved performance.

The zones of high air permeability, such as the air passageways, are configured to enhance the breathability of the article to reduce the hydration of the wearer's skin during use without allowing excessive condensation of vapor, such as urine, on the garment facing surface of the outer cover 20. Such condensation of vapor on the outer surface of the diaper 10 can undesirably dampen the wearer's clothes. The zones of high air permeability are generally located in the area of the diaper over which air and vapor can transfer from the bodyside liner 22, through the absorbent body 24 and any other intervening layer or layers of material, and out the vapor permeable outer cover 20. For example, the zones of high air permeability may be located throughout the entire absorbent body 24 or may be selectively located in those regions of the absorbent body 24 that provide the maximum air exchange, such as the intermediate section 16 of the diaper 20. In a particular aspect, the zones of high air permeability are located in the front and intermediate sections 12 and 16, respectively, of the diaper 10 for improved air exchange.

The zones of high absorption, on the other hand, are not designed to transfer a high level of air and vapor from the interior of the diaper 10. Thus, the air exchange from the bodyside liner 22 of the diaper 10 to the outer cover 20 of the diaper and into the ambient atmosphere (exterior of the diaper 10) occurs generally through the absorbent body 24 in the zones of high air permeability. Some air exchange through the absorbent body 24 can also occur in the zones of high absorption to a limited degree. The zones of high air permeability may have any desired configuration including rectangular, circular, hourglass, oval, and the like, and may also include selected longitudinal or lateral strips or multiple regions which may be intermittently located.

The zones of high air permeability may have any desired dimensions that effectively provide improved air exchange while preventing excessive condensation of vapor from the absorbent body 24 through and onto the garment facing surface of the outer cover 20. Desirably, the zones of high air permeability may define a total area of from about 5 to about 75 percent, more desirably at least about 10 percent, even more desirably from about 10 to about 70 percent, and still more desirably from about 10 to about 60 percent of the total surface area of the absorbent body 24 of the diaper 10. For example, in a diaper 10 intended for use on a medium sized infant, the zones of high air permeability may define a total area of from about 6 to about 90 square centimeters.

When the total area of the zones of high air permeability is greater than the above amounts, the diaper 10 may exhibit an undesirable amount of condensation of vapor on the exposed, garment facing surface of the outer cover 20 undesirably resulting in a clammy feeling on the outer surface of the diaper 10. Whereas, when the total area of the zones of high air permeability is less than the above amounts, the diaper 10 may exhibit a low level of air exchange resulting in high levels of skin hydration that can undesirably lead to skin irritation and rash.

The zones of high air permeability of the absorbent body 24 of the diaper 10 are constructed to be substantially permeable to at least air and preferably permeable to water vapor. For example, the zones of high air permeability of the absorbent body 24 define a Frazier Porosity value which is at least about 10 percent, more desirably at least about 20 percent and even more desirably at least about 50 percent greater than the Frazier Porosity value of the zones of high absorption of the absorbent body 24. As used herein, the term "Frazier Porosity" refers to the value determined according to the Frazier Porosity Test described in U.S. Patent 6,217,890 issued April 17, 2001 to Paul et al. When the zones of high air permeability exhibit Frazier Porosity values less than those indicated above, the diaper 10 may exhibit a low level of air exchange resulting in high levels of skin hydration that can undesirably lead to skin irritation and rash.

The zones of high air permeability may be provided in a variety of ways. The zones of high air permeability may be integral portions of the absorbent body 24 of the absorbent article or may be provided by apertures, holes or open spaces in the absorbent body 24. For example, portions of the absorbent body 24 may be discontinuous or removed to provide the zones. Alternatively, the zones of high air permeability may be provided by portions of the absorbent body 24 that are constructed to absorb less fluid exudates thereby resulting in improved air flow through such portions in use. For example, portions of the absorbent body 24 may be void of or contain substantially less high-absorbency material than other portions of the absorbent body 24 to provide such improved air flow. Portions of the absorbent body 24 may otherwise be treated or coated with a solution that renders them hydrophobic to provide the zones of high air permeability in selected areas. In other alternative configurations, the zones of high air permeability may be provided by creating voids or holes in the absorbent body 24 and placing other materials having a higher air permeability than the absorbent body 24, such as those materials described below as being suitable for the surge management layer 34, in the holes or voids.

Due to the thinness of absorbent body 24 and the high absorbency material within the absorbent body 24, the liquid uptake rates of the absorbent body 24, by itself, may be too low, or may not be adequately sustained over multiple insults of liquid into the absorbent body 24. To improve the overall liquid uptake and air exchange, the diaper 10 of the different aspects of the present invention may further include a porous, liquid-permeable layer of surge management material 34, as representatively illustrated in Fig. 1. The surge management layer 34 is typically less hydrophilic than the absorbent body 24, and has an operable level of density and basis weight to quickly collect and temporarily hold liquid surges, to transport the liquid from its initial entrance point and to substantially completely release the liquid to other parts of the absorbent body 24. This configuration can help prevent the liquid from pooling and collecting on the portion of the diaper 10 positioned against the wearer's skin, thereby reducing the feeling of wetness by the wearer. The structure of the surge management layer 34 also generally enhances the air exchange within the diaper 10.

Various woven and nonwoven fabrics can be used to construct the surge management layer 34. For example, the surge management layer 34 may be a layer composed of a meltblown or spunbond web of synthetic fibers, such as polyolefin fibers. The surge management layer 34 may also be a bonded-carded-web or an airlaid web composed of natural and synthetic fibers. The bonded-carded-web may, for example, be a thermally bonded web that is bonded using low melt binder fibers, powder or adhesive. The webs can optionally include a mixture of different fibers. The surge management layer 34 may be composed of a substantially hydrophobic material, and the hydrophobic material may optionally be treated with a surfactant or otherwise processed to impart a desired level of wettability and hydrophilicity. In a particular aspect, the surge management layer 34 includes a hydrophobic, nonwoven material having a basis weight of from about 30 to about 120 grams per square meter.

For example, in a particular aspect, the surge management layer 34 may include a bonded-carded-web, non-woven fabric that includes bicomponent fibers and that defines an overall basis weight of about 83 grams per square meter. The surge management layer 34 in such a configuration can be a homogeneous blend composed of about 60 weight percent polyethylene/polyester (PE/PET), sheath-core bicomponent fibers that have a fiber denier of about 3.33 dtex (3d) and about 40 weight percent single component polyester fibers that have a fiber denier of about 6.66 dtex (6d) and that have fiber lengths of from about 3.8 to about 5.08 centimeters.

In the illustrated aspects, the surge management layer 34 is arranged in a direct, contacting liquid communication with the absorbent body 24. The surge management layer 34 may be operably connected to the bodyside liner 22 with a conventional pattern of adhesive, such as a swirl adhesive pattern. In addition, the surge management layer 34 may be operably connected to the absorbent body 24 with a conventional pattern of adhesive. The amount of adhesive add-on should be sufficient to provide the desired levels of bonding, but should be low enough to avoid excessively restricting the movement of liquid from the bodyside liner 22, through the surge management layer 34 and into the absorbent body 24.

The absorbent body 24 is positioned in liquid communication with surge management layer 34 to receive liquids released from the surge management layer 34, and to hold and store the liquid. In the shown aspect, the surge management layer 34 includes a separate layer that is positioned over another, separate layer including the absorbent body 24, thereby forming a dual-layer arrangement. The surge management layer 34 serves to quickly collect and temporarily hold discharged liquids, to transport such liquids from the point of initial contact and spread the liquid to other parts of the surge management layer 34, and then to substantially completely release such liquids into the layer or layers constituting the absorbent body 24.

The surge management layer 34 can be of any desired shape. Suitable shapes include for example, circular, rectangular, triangular, trapezoidal, oblong, dog-boned, hourglass-shaped, or oval. In certain aspects, for example, the surge management layer 34 can be generally rectangular-shaped. In the illustrated aspects, the surge management layer 34 is coextensive with the absorbent body 24. Alternatively, the surge management layer 34 may extend over only a part of the absorbent body 24. Where the surge management layer 34 extends only partially along the length of the absorbent body 24, the surge management layer 34 may be selectively positioned anywhere along the absorbent body 24. For example, the surge management layer 34 may function more efficiently when it is offset toward the front waist section 12 of the diaper 10. The surge management layer 34 may also be approximately centered about the longitudinal center line of the absorbent body 24.

Additional materials suitable for the surge management layer 34 are set forth in U.S. Patent No. 5,486,166 issued January 23, 1996 in the name of C. Ellis et al. and entitled "FIBROUS NONWOVEN WEB SURGE LAYER FOR PERSONAL CARE ABSORBENT ARTICLES AND THE LIKE"; U.S. Patent No. 5,490,846 issued February 13, 1996 in the name of Ellis et al. and entitled "IMPROVED SURGE MANAGEMENT FIBROUS NONWOVEN WEB FOR PERSONAL CARE ABSORBENT ARTICLES AND THE LIKE"; and U.S. Patent No. 5,364,382 issued November 15, 1994 in the name of Latimer et al. and entitled "ABSORBENT STRUCTURE HAVING IMPROVED FLUID SURGE MANAGEMENT AND PRODUCT INCORPORATING SAME" .

As representatively illustrated in Fig. 1, the diaper 10 may also include a ventilation layer 32 located between the outer cover 20 and the absorbent body 24. The ventilation layer 32 serves to facilitate the movement of air within and through the diaper 10 and prevent the outer cover 20 from being in surface to surface contact with at least a portion of the absorbent body 24. Specifically, the ventilation layer 32 serves as a conduit through which air and water vapor can move from the absorbent body 24 through the vapor permeable outer cover 20.

The ventilation layer 32 may be formed from materials described above as being suitable for the surge management layer 34 such as nonwoven, (e.g., spunbond, meltblown or carded), woven, or knitted fibrous webs composed of natural fibers and/or synthetic polymeric fibers. Suitable fibers include, for example, acrylic fibers, polyolefin fibers, polyester fibers, or blends thereof. The ventilation layer 32 may also be formed from a porous foam material such as an open-celled polyolefin foam, a reticulated polyurethane foam, and the like. The ventilation layer 32 may include a single layer of material or a composite of two or more layers of material. In a particular aspect, the ventilation layer 32 includes a hydrophobic, nonwoven material having a thickness of at least about 0.10 centimeters determined under a restraining pressure of 0.05 psi (0.34 kPa) and a basis weight of from about 20 to about 120 grams per square meter. For example, the ventilation layer 32 may include a bonded-carded-web, nonwoven fabric that includes bicomponent fibers and that defines an overall basis weight of about 83 grams per square meter. The ventilation layer 32 in such a configuration can be a homogeneous blend composed of about 60 weight percent polyethylene/polyester (PE/PET), sheath-core bicomponent fibers that have a fiber denier of about 3.33 dtex (3d) and about 40 weight percent single component polyester fibers that have a fiber denier of about 6.66 dtex (6d) and that have fiber lengths of from about 3.8 to about 5.08 centimeters.

The ventilation layer 32 can be of any desired shape. Suitable shapes include for example, circular, rectangular, triangular, trapezoidal, oblong, dog-boned, hourglass-shaped, or oval. The ventilation layer 32 may extend beyond, completely over or partially over the absorbent body 24. For example, the ventilation layer 32 may suitably be located over the intermediate section 16 of the diaper 10 and be substantially centered side-to-side with respect to the longitudinal centerline 36 of the diaper 10. It is generally desired that the entire absorbent body 24 be overlaid with the ventilation layer 32 to prevent substantially all surface to surface contact between the outer cover 20 and the absorbent body 24. In the illustrated aspects, the ventilation layer 32 is coextensive with the absorbent body 24. This allows for the maximum degree of air exchange with minimal dampness on the garment facing surface of the outer cover 20.

In the illustrated aspects, the ventilation layer 32 is arranged in a direct, contacting liquid communication with the absorbent body 24. The ventilation layer 32 may be operably connected to the outer cover 20 with a conventional pattern of adhesive, such as a swirl adhesive pattern. In addition, the ventilation layer 32 may be operably connected to the absorbent body 24 with a conventional pattern of adhesive. The amount of adhesive add-on should be sufficient to provide the desired levels of bonding, but should be low enough to avoid excessively restricting the movement of air and vapor from the absorbent body 24 and through the outer cover 20.

The ventilation layer 32 may further serve to quickly collect and temporarily hold discharged liquids, which pass through the absorbent body 24 and, in particular, through the zones of high air permeability within the absorbent body 24. The ventilation layer 32 may then transport such liquids from the point of initial contact and spread the liquid to other parts of the ventilation layer 32, and then substantially completely release such liquids into the layer or layers of the absorbent body 24.

In order to improve the skin health of wearers of absorbent articles, a skin care composition is applied to the bodyfacing surface 11 of the bodyside liner 22 of the diaper 10. The skin care composition generally can include emollient(s) and viscosity enhancer(s). The composition can also include natural fats or oils, solidifying agents and sterols or sterol derivatives. For example, the skin care compositions of the invention may include from about 40 to about 95 percent by weight of one or more emollients; and from about 5 to about 60 percent by weight of one or more viscosity enhancers. The skin care composition may include other ingredients as well. Ranges are used to describe the relative amounts of components in the compositions of the invention as well as to describe the relative physical properties of the compositions. These ranges are illustrative and one of skill in the art will recognize that the nature of the composition will dictate the various levels of components that must be used to achieve the intended benefit for the skin barrier. The levels can be determined by routine experimentation in view of the disclosure provided herein.

The skin care compositions of the invention can be in a variety of physical forms including emulsions, lotions, creams, ointments, salves, suspensions, gels or hybrids of these forms.

The emollients of the skin care compositions act as lubricants to reduce the abrasiveness of the bodyside liner 22 to the skin and, upon transfer to the skin, help to maintain the soft, smooth and pliable appearance of the skin. In general, emollients are skin-conditioning ingredients that help to soften, smooth, plasticize, lubricate, moisturize, improve the appearance of, improve the feel of and protect skin. Suitable emollients that can be incorporated into the compositions include, but are not limited to, oils such as petroleum based oils, petrolatum, vegetable based oils, hydrogenated vegetable oils, animal oils, hydrogenated animal oils, mineral oils, natural or synthetic oils, alkyl dimethicones, alkyl methicones, alkyldimethicone copolyols, phenyl silicones, siliconized waxes, alkyl trimethylsilanes, dimethicone, lanolin and its derivatives, esters, branched esters, glycerol esters and derivatives, propylene glycol esters and derivatives, alkoxylated carboxylic acids, alkoxylated alcohols, fatty alcohols, triglycerides, alkyl hydroxystearates and mixtures of such compounds. The esters can be selected from cetyl palmitate, stearyl palmitate, cetyl stearate, isopropyl laurate, isopropyl myristate, isopropyl palmitate, behenyl behenate, stearyl behenate, $C_{12}$-$C_{15}$ alkyl fumarate, $C_{20}$-$C_{40}$ alkyl behenate, dibehenyl fumarate, branched esters and mixtures thereof. Ethers such as eucalyptol, cetearyl glucoside, dimethyl isosorbicide polyglyceryl-3 cetyl ether, polyglyceryl-3 decyltetradecanol, propylene glycol myristyl ether and mixtures thereof can also be used as emollients. The fatty alcohols include octyldodecanol, lauryl, myristyl, cetyl, stearyl and behenyl alcohol, $C_{24}$ and greater fatty alcohols and mixtures thereof. For example, a particularly well suited emollient is petrolatum. Other conventional emollients may also be added in a manner that maintains the desired properties of the skin care compositions set forth herein.

To provide improved stability and transfer to the skin of the wearer, the skin care compositions may include from about 40 to about 95 percent by weight, desirably from about 50 to about 85 percent by weight, and more desirably from about 60 to about 75 percent by weight of the emollient. In particular aspects, the emollient can be at least a minimum of about 40 percent by weight. The emollient can alternatively be at least about 50 percent, and optionally, can be at least about 60 percent to provide improved performance. In other aspects, the emollient can be not more than a maximum of about 95 percent by weight. The emollient can alternatively be not more than about 85 percent, and optionally, can be not more than about 75 percent to provide improved effectiveness. Compositions that include an amount of emollient greater than the recited amounts tend to have lower viscosities that undesirably lead to migration of the composition. Whereas, compositions that include an amount of emollient less than the recited amounts tend to provide less transfer to the wearer's skin.

One or more viscosity enhancers may be added to the skin care composition to increase the viscosity, to help stabilize the composition on the bodyfacing surface 11 of the bodyside liner 22 and, thereby, to reduce migration and improve transfer to the skin. The viscosity enhancer increases the meltpoint viscosity of the compositions to have a high viscosity (greater than about 50,000 centipoise) under low shear at the "hot box car" stability temperature of about 54.5°C and at lower temperatures. Having viscosity at elevated temperatures prevents the compositions from migrating into or away from the materials to which they are applied. However, the viscosity enhancer component also provides a low viscosity (less than about 5,000 centipoise) under shear for the compositions at process conditions. Typically, process temperatures are approximately 5°C above the melting point of the composition. Generally, the process temperature is about 60°C or higher. Different skin care compositions of the invention will have different melting points. The viscosity enhancers of the invention are capable of maintaining the viscosity of skin care compositions of the invention up to temperatures just below the desired processing temperature for a given composition.

Examples of suitable viscosity enhancers include, but are not limited to, polyolefin resins, lipophilic/oil thickeners, ethylene/vinyl acetate copolymers, natural clays, synthetic analogs of natural clays, organically modified clays, quaternary modified clays, quaternary starch compounds, polyethylene, silica, silica silylate, silica methyl silylate, colloidal silicone dioxide, cetyl hydroxy ethyl cellulose, other organically modified celluloses, PVP/decane copolymer, PVM/MA decadiene crosspolymer, PVP/eicosene copolymer, PVP/hexadecane copolymer, microcrystalline wax, hexadecyl-cosanyl-hexacosanate, shellac wax, glycol montanate, PEG-12 carnauba, synthetic paraffin, ozokerite, $C_{20}$-$C_{40}$ alkyl hydroxystearyl stearate, polyperfluoromethylisopropylether montan wax, magnesium aluminum silicate, polymethacrylate polymers, polystyrene copolymers and mixtures of these compounds. A particularly well suited viscosity enhancer is an ethylene/vinyl acetate copolymer commercially available from E. I. Dupont de Nemours under the trade designation "ELVAX". Additionally, the compounds identified herein as suitable solidifying agents may also function as viscosity enhancers to benefit the rheology of the compositions of the invention.

To provide the improved transfer to the skin of the wearer, the skin care composition may include from about 5 to about 60 percent by weight, desirably from about 15 to about 40 percent by weight, and more desirably from about 20 to about 35 percent by weight of the viscosity enhancer for reduced migration and improved transfer to the wearer's skin.). In particular aspects, the viscosity enhancer can be at least a minimum of about 5 percent by weight. The viscosity enhancer can alternatively be at least about 15 percent, and optionally, can be at least about 20 percent to provide improved performance. In other aspects, the viscosity enhancer can be not more than a maximum of about 60 percent by weight. The viscosity enhancer can alternatively be not more than about 40 percent, and optionally, can be not more than about 35 percent to provide improved effectiveness.

The solidifying agent(s) in the skin care compositions of the present invention primarily functions to solidify the composition so that the composition is a solid at room temperature and has a penetration hardness of at least 5 mm and has a melting point of at least 32°C. The solidifying agent may also provide a tackiness to the skin care composition that improves the transfer by adhesion to the skin of the wearer. Depending on the solidifying agent selected, the solidifying agent can also modify the mode of transfer so that the skin care composition tends to fracture or flake off instead of actually rubbing off onto the skin of the wearer which can lead to improved transfer to the skin. The solidifying agent may further function as an emollient, occlusive agent, moisturizer, barrier enhancer, viscosity enhancer and combinations thereof. The solidifying agents may include waxes as well as compounds that perform functionally as waxes.

The solidifying agents can be selected from alkyl siloxanes, polymers, hydrogenated vegetable oils having a melting point of 35°C or greater, fatty acid esters and branched esters with a melting point of 35°C or greater, alkyl hydroxystearates (>C16), alkoxylated alcohols and alkoxylated carboxylic acid. Additionally, the solidifying agents can be selected from animal, vegetable and mineral waxes, synthetic waxes and alkyl silicones. Examples of solidifying agents include, but are not limited to, the following: alkyl silicones, alkyl trimethylsilanes, beeswax, behenyl behenate, behenyl benzoate, $C_{24}$-$C_{28}$ alkyl dimethicone, $C_{30}$ alkyl dimethicone, cetyl methicone, stearyl methicone, cetyl dimethicone, stearyl dimethicone, cerotyl dimethicone, candelilla wax, carnauba, synthetic carnauba, PEG-12 carnauba, cerasin, hydrogenated microcrystalline wax, jojoba wax, microcrystalline wax, lanolin wax, ozokerite, paraffin, synthetic paraffin, cetyl esters, behenyl behenate, $C_{20}$-$C_{40}$ alkyl behenate, $C_{12}$-$C_{15}$ lactate, cetyl palmitate, stearyl palmitate, isosteryl behenate, lauryl behenate, stearyl benzoate, behenyl isostearate, cetyl myristate, cetyl octanoate, cetyl oleate, cetyl ricinoleate, cetyl stearate, decyl oleate, di-$C_{12}$-$C_{15}$ alkyl fumerate, dibehenyl fumerate, myristyl lactate, myristyl lignocerate, myristyl myristate, myristyl stearate, lauryl stearate, octyldodecyl stearate, octyldodecyl stearoyl stearate, oleyl arachidate, oleyl stearate, tridecyl behenate, tridecyl stearate, tridecyl stearoyl stearate, pentaerythrityl tetrabehenate, pentaerythritylhydrogenated rosinate, pentaerythrityl distearate, pentaerythrityl tetraabeite, pentaerythrityl tetracocoate, pentaerythrityl tetraperlargonate, pentaerythrityl tetrastearate, ethylene vinyl acetate, polyethylene, hydrogenated cottonseed oil, hydrogenated vegetable oil, hydrogenated squalene, hydrogenated coconut oil, hydrogenated jojoba oil, hydrogenated palm oil, hydrogenated palm kernel oil, hydrogenated olive oil, polyamides, metal stearates and other metal soaps, $C_{30}$-$C_{60}$ fatty alcohols, $C_{20+}$ fatty amides, polypropylene, polystyrene, polybutane, polybutylene terephthalate, polydipentane, polypropylene, zinc stearate, dodecyl laurate, stearyl palmitate, octadecyl hexadecanoate, octadecyl palmitate, stearyl behenate, docosyl octanoate, tetradecyl-octadecanyl behenate, hexadecyl-cosanyl hexacosanate, shellac wax, glycol montanate, fluoranated waxes, $C_{20}$-$C_{40}$ alkyl hydroxystearyl stearate and mixtures of such compounds. Suitable branched esters include tetradecyl-octadecanyl behenate and hexadecyl-cosanyl-hexacosanate. In one aspect, the solidifying agent is a blend including about 70 weight percent cerasin wax, about 10 weight percent microcrystalline wax, about 10 weight percent paraffin wax and about 10 weight percent cetyl esters (synthetic spermaceti wax).

To provide improved transfer to the skin of the wearer, the composition may include from about 5 to about 55 percent by weight, desirably from about 15 to about 45 percent by weight, and more desirably from about 25 to about 35 percent by weight of solidifying agent(s). In particular aspects, the solidifying agent can be at least a minimum of about 5 percent by weight. The solidifying agent can alternatively be at least about 15 percent, and optionally, can be at least about 25 percent to provide improved performance. In other aspects, the solidifying agent can be not more than a maximum of about 55 percent by weight. The solidifying agent can alternatively be not more than about 45 percent, and optionally, can be not more than about 35 percent to provide improved effectiveness. Compositions that include an amount of solidifying agent less than the recited amounts tend to be too soft and may have lower viscosities that may undesirably lead to migration of the composition away from bodyfacing surfaces 11 of the absorbent article, thus diminishing transfer to the wearer's skin. Whereas, compositions that include an amount of solidifying agent greater than the recited amounts tend to provide less transfer to the wearer's skin.

In another aspect, the skin care compositions of the invention include fats and oils that provide a source of essential and non-essential fatty acids similar to those found in the skin's natural barrier. Fats and oils include compounds that are fats, oils, essential oils, fatty acids, fatty alcohols, phospholipids and mixtures of such compounds. Fats and oils include oils derived from plant and animal sources. Similarly, the essential oils include essential oils derived from plant sources. Those of skill in the art would understand that all compounds commonly understood to have the structure of or to function as fats, oils, essential oils, fatty acids, fatty alcohols and phospholipids can be used as the natural fat or oil component of the composition of the invention. While an exhaustive list of each and every fat and oil that could be used in the compositions of the invention is not provided, those of skill in the art will understand and appreciate the individual compounds that can serve as a fat or oil component of the compositions of the invention.

Representative examples of fats and oils include, but are not limited to: Avocado Oil, Apricot Oil, Babassu Oil, Borage Oil, Camellia Oil, Canola Oil, Castor Oil, Coconut Oil, Corn Oil, Cottonseed Oil, Evening Primrose Oil, Hydrogenated Cottonseed Oil, Hydrogenated Palm Kernel Oil, Maleated Soybean Oil, Meadowfoam Oil, Palm Kernel Oil, Peanut Oil, Rapeseed Oil, Safflower Oil, Sphingolipids, Sweet Almond Oil, Tall Oil, Lanolin, Lanolin Alcohol, Lauric Acid, Palmitic Acid, Stearic Acid, Linoleic Acid, Stearyl Alcohol, Lauryl Alcohol, Myristyl Alcohol, Behenyl Alcohol, Rose Hip Oil, Calendula Oil, Chamomile Oil, Eucalyptus Oil, Juniper Oil, Sandlewood Oil, Tea Tree Oil, Sunflower Oil, and Soybean Oil. Another suitable fat/oil for the compositions of the invention is PROLIPID 141 blend available from International Specialty Products of Wayne, New Jersey. The PROLIPID 141 blend is a mixture of glyceryl stearate, fatty acids, fatty alcohols and phospholipids.

In order to assist in replenishing skin barrier enhancing agents, the compositions of the invention may include fats and oils in an amount of from about 0.1 to about 55 percent by weight, desirably from about 5 to about 40 percent by weight, and more desirably from about 15 to about 30 percent by weight of the composition. In particular aspects, the fats and oils can be at least a minimum of about 0.1 percent by weight. The fats and oils can alternatively be at least about 5 percent, and optionally, can be at least about 15 percent to provide improved performance. In other aspects, the fats and oils can be not more than a maximum of about 55 percent by weight. The fats and oils can alternatively be not more than about 40 percent, and optionally, can be not more than about 30 percent to provide improved effectiveness.

The skin care compositions of the invention also include sterols and sterol derivatives that act in combination with the natural fats/oils to provide natural skin barrier enhancement and skin barrier recovery. Sterols and sterol derivatives that can be used in the compositions of the invention include, but are not limited to: β-sterols having a tail on the 17 position and having no polar groups for example, cholesterol, sitosterol, stigmasterol, and ergosterol, as well as, $C_{10}$-$C_{30}$ cholesterol/lanosterol esters, cholecalciferol, cholesteryl hydroxystearate, cholesteryl isostearate, cholesteryl stearate, 7-dehydrocholesterol, dihydrocholesterol, dihydrocholesteryl octyldecanoate, dihydrolanosterol, dihydrolanosteryl octyldecanoate, ergocalciferol, tall oil sterol, soy sterol acetate, lanasterol, soy sterol, avocado sterols, "AVOCADIN" (trade name of Croda Ltd of Parsippany, New Jersey), sterol esters and similar compounds, as well as mixtures thereof. The compositions of the invention can include sterols, sterol derivatives or mixtures of both sterols and sterol derivatives in an amount of from about 0.1 to about 10 percent by weight, desirably from about 0.5 to about 5 percent by weight and more desirably from about 0.8 to about 1 percent by weight of the composition. In particular aspects, the sterols can be at least a minimum of about 0.1 percent by weight. The sterols can alternatively be at least about 0.5 percent, and optionally, can be at least about 0.8 percent to provide improved performance. In other aspects, the sterols can be not more than a maximum of about 10 percent by weight. The sterols can alternatively be not more than about 5 percent, and optionally, can be not more than about 1 percent to provide improved effectiveness.

If it is desired that the skin care composition provide a treatment for the skin, the skin care composition can also include an active ingredient such as a diaper rash skin protectant. Skin protectants are drug products that protect injured or exposed skin or mucous membrane surface from harmful or annoying stimuli. Suitable active ingredients, in addition to those mentioned above as suitable emollients, that can be incorporated into the composition include, but are not limited to, allantoin and its derivatives, aloe, aluminum hydroxide gel, calamine, cocoa butter, dimethicone, cod liver oil, glycerin, kaolin and its derivatives, lanolin and its derivatives, mineral oil, petrolatum, shark liver oil, talc, topical starch, zinc acetate, zinc carbonate, zinc oxide and the like, and mixtures thereof. The skin care composition may include from about 0.10 to about 55 percent by weight of the active ingredient depending upon the skin protectant, the amount desired to be transferred to the skin or the amount of a particular skin protectant required in the U.S. Food and Drug Administration monograph.

In order to better enhance the benefits to the wearer, additional ingredients can be included in the skin care compositions of the present invention. For example, the classes of ingredients that may be used and their corresponding benefits include, without limitation: antifoaming agents (reduce the tendency of foaming during processing); antimicrobial actives; antifungal actives; antiseptic actives; antioxidants (product integrity); antioxidants-cosmetic (reduce oxidation); astringents-cosmetic (induce a tightening or tingling sensation on skin); astringent-drug (a drug product that checks oozing, discharge, or bleeding when applied to skin or mucous membrane and works by coagulating protein); biological additives (enhance the performance or consumer appeal of the product); colorants (impart color to the product); deodorants (reduce or eliminate unpleasant odor and protect against the formation of malodor on body surfaces); other emollients (help to maintain the soft, smooth, and pliable appearance of the skin by their ability to remain on the skin surface or in the stratum corneum to act as lubricants, to reduce flaking, and to improve the skin's appearance); external analgesics (a topically applied drug that has a topical analgesic, anesthetic, or antipruritic effect by depressing cutaneous sensory receptors, or that has a topical counterirritant effect by stimulating cutaneous sensory receptors); film formers (to hold active ingredients on the skin by producing a continuous film on skin upon drying); fragrances (consumer appeal);

silicones/organomodified silicones (protection, water resistance, lubricity, softness); oils (mineral, vegetable, and animal); natural moisturizing agents (NMF) and other skin moisturizing ingredients known in the art; opacifiers (reduce the clarity or transparent appearance of the product); powders (enhance lubricity, oil adsorption, provide skin protection, astringency, opacity, etc.); skin conditioning agents; solvents (liquids employed to dissolve components found useful in the cosmetics or drugs); and surfactants (as cleansing agents, emulsifying agents, solubilizing agents, and suspending agents).

An important property of the skin care compositions of the different aspects of the present invention is their ability to remain on the surface of the bodyside liner 22 and their resistance to migration into the diaper 10 such that they can readily be transferred to the wearer's skin. In this regard, the articles having the skin care compositions of the present invention applied to their bodyside liner 22 define a z-direction migration loss of no more than about 55%, desirably no more than about 50%, more desirably no more than about 45%, even more desirably no more than about 40% and yet even more desirably no more than about 35% when subjected to the Z-Direction Lotion Migration Test described in U.S. Patent 6,149,934 issued November 21, 2000 to Krzysik et al.

In articles that have a greater z-direction migration loss, the composition undesirably migrates into the interior and along the surface of the bodyside liner 22 and at times through the bodyside liner 22 into the absorbent body 24 of the article which results in a lower reduction in abrasion and less transfer to the skin of the wearer.

Moreover, to provide the improved stability and transfer to the skin of the wearer, the skin care compositions of the present invention may define a melting point of from about 32°C to about 100°C, desirably from about 35°C to about 80°C, and more desirably from about 40°C to about 75°C. Compositions that have lower melting points exhibit migration of the composition during use and at elevated temperatures in storage that can undesirably result in reduced transfer to the skin. Whereas, compositions that have higher melting points may require that the composition be at a temperature above the flash point of the bodyside liner 22 material which can undesirably lead to fires. The melting points of the skin care compositions of the invention cause the compositions to be relatively immobile and localized on the bodyfacing surface 11 of the diaper 10 at room temperature and readily transferable to the skin of the wearer at body temperatures. However, the skin care compositions of the invention are not completely liquid under extreme storage conditions. Desirably, the skin care compositions are easily transferable to the skin by way of normal contact, wearer motion, adhesion or body heat. When the skin care compositions are relatively immobilized at room temperature, a lesser quantity of composition is required on the bodyfacing surface 11 to provide a beneficial effect.

The skin care composition of the present invention may further define a low shear viscosity at about 55°C of greater than about 50,000 centipoise, desirably from about 50,000 to about 1,000,000 centipoise, and more desirably from about 100,000 to about 800,000 centipoise for reduced migration and improved transfer to the skin of the wearer. Compositions that have lower melt point viscosities exhibit migration of the composition through the bodyside liner 22 into the absorbent body 24 of the article which can undesirably result in reduced transfer to the skin. Whereas, compositions that have higher melt point viscosities may be so solid as to also exhibit a reduced transfer to the skin.

Further, to provide the improved stability and transfer to the skin of the wearer, the skin care compositions of the present invention may also define a high shear viscosity of less than about 5,000 centipoise, desirably from about 100 to about 500 centipoise, and more desirably from about 150 to about 250 centipoise at a temperature of about 60°C (or higher temperatures depending on the components and melting point of the composition).

The penetration hardness of the skin care compositions of this invention can be from about 5 to about 365 millimeters, more desirably from about 10 to about 300 millimeters, more desirably from about 20 to about 200 millimeters, and still more desirably from about 40 to about 120 millimeters at 25°C. (Compositions having a needle penetration hardness greater than 365 millimeters cannot be measured using ASTM method D 1321). The hardness of the skin care compositions of this invention is important for two reasons. First, the softer the formulation the more mobile the formulation will be, making the formulation more likely to migrate to the inner plies of the diaper 10, which is not desirable. Secondly, softer compositions tend to be more greasy/oily to the touch, which is also less desirable.

The skin care composition may be applied to the entire bodyfacing surface 11 of the bodyside liner 22 or may be selectively applied to particular sections of the bodyfacing surface 11, such as the medial section along the longitudinal centerline of the diaper 10, to provide greater lubricity of such sections and to transfer such composition to the wearer's skin. Alternatively, the bodyfacing surface 11 of the bodyside liner 22 may include multiple stripes of the skin care composition applied thereto. For example, the bodyfacing surface 11 of the bodyside liner 22 may include from 1 to 20 stripes of skin care composition extending along the longitudinal direction of the diaper 10. The stripes may extend the full length of the bodyside liner 22 or only a portion thereof. The stripes may also define a width of from about 0.2 to about 1 centimeters.

The skin care composition should cover a sufficient amount of the bodyfacing surface 11 of the bodyside liner 22 to ensure adequate transfer to the skin and reduced abrasion between the bodyside liner 22 and the wearer's skin. Desirably, the skin care composition is applied to at least about 5 percent and more desirably at least about 25 percent of the bodyfacing surface 11 of the bodyside liner 22.

The skin care composition can be applied to the bodyside liner 22 at any add-on level that provides the desired transfer benefit. For example, the total add-on level of the skin care composition can be from about 0.05 to about 100

mg/cm$^2$, desirably from about 1 to about 50 mg/cm$^2$ and more desirably from about 10 to about 40 mg/cm$^2$ for improved performance. The add-on amount will depend upon the desired effect of the skin care composition on the skin barrier function and the specific composition. As discussed above, the improved stability and reduced tendency to migrate of the skin care compositions of the present invention allows a lesser amount of skin care composition to be applied to the bodyside liner 22 to achieve the same benefit when compared with conventional compositions.

The skin care composition may be applied to the bodyside liner 22 in any of many well known manners. A preferred method to uniformly apply the skin care composition to the bodyfacing surface 11 of the bodyside liner 22 is spraying or slot coating. Spraying or slot coating the skin care composition is the most exact process and offers maximum control of the skin care composition distribution and transfer rate. However, other methods, such as roto-gravure or flexographic printing and foam application can be used. The skin care compositions of the present invention can be applied after the bodyfacing material has been incorporated into the absorbent article or prior to incorporating the body facing material into the absorbent article.

The skin care composition may be applied to the bodyside liner 22 by (a) heating the skin care composition to a temperature above the melting point of the skin care composition, causing the skin care composition to melt, (b) uniformly applying the melted skin care composition to the bodyfacing surface 11 of the bodyside liner 22; and (c) resolidifying the skin care composition applied to the bodyfacing surface 11. Desirably, resolidification of the skin care composition occurs almost instantaneously, without the need for external cooling devices such as chill rolls. This can occur if the skin care composition is heated to a temperature only slightly above or at the melting point of the skin care composition. However, external cooling devices such as chill rolls, either before or after the application of melt, can be used if desired to accelerate resolidification. Other cooling methods such as cooling tunnels could also be used.

The increased viscosity of the skin care composition at the process temperature and the instantaneous resolidification tends to impede penetration of the composition into the bodyside liner 22 and absorbent body 24 of the diaper 10 and retain it on the bodyfacing surface 11 of the bodyside liner 22, which is advantageous. For example, the temperature of the melted skin care composition can advantageously be less than about 20°C, more desirably less than about 10°C, and still more desirably less than about 5°C above the melting point of the skin care composition prior to applying it to the bodyside liner 22 for reduced migration. As the temperature of the melted skin care composition approaches the freezing point of the skin care composition, the viscosity of the melted skin care composition generally increases, which further enhances the tendency of the melted skin care composition to be retained on the bodyfacing surface 11.

The systems of the invention also include a wet wipe having a skin care solution. The wet wipes of the invention can be generally rectangular (including generally square) in shape and may have any suitable unfolded width and length. For example, the wet wipe may have an unfolded length of from about 2.0 to about 80.0 centimeters and desirably from about 10.0 to about 25.0 centimeters and an unfolded width of from about 2.0 to about 80.0 centimeters and desirably from about 10.0 to about 25.0 centimeters. Typically, each individual wet wipe is arranged in a folded configuration and stacked one on top of the other to provide a stack of wet wipes. Such folded configurations are well known to those skilled in the art and include c-folded, z-folded, quarter-folded configurations and the like. The stack of folded wet wipes may be placed in the interior of a container, such as a plastic tub or flexible refill bag, to provide a package of wet wipes for eventual sale to the consumer. Alternatively, the wet wipes may include a continuous strip of material which has perforations between each wipe and which may be arranged in a stack or wound into a roll for dispensing.

The wet wipe or wipe-type product of the systems of the invention may assume a variety of shapes, including but not limited to, generally circular, oval, square, or irregularly shaped. The size of the wet wipe or wipe-type product will also vary depending upon the desired end use of the wipe.

The materials of the basesheet, single or multi-layered, of the wet wipe or the wipe-type product of the systems of the present invention may be varied to provide different physical properties. The different physical properties which a layer may be configured to provide by selecting the appropriate materials include softness, resiliency, strength, flexibility, integrity, toughness, absorbency, liquid retention, thickness, tear resistance, surface texture, drapability, hand, wettability, wicking ability and similar properties and combinations thereof. The wipe can be configured to provide all desired physical properties within one layer or configured to provide only specific physical properties within individual layers of a multi-layered wipe. For example, the wet wipes may include at least one layer of material that is configured to provide strength and resilience to the wet wipe and at least one other layer which is configured to provide a soft, gentle wiping surface to the wet wipe. Desirably, the wet wipes provide a soft wiping surface for contact with the skin.

The layer or layers of the wet wipe or wipe-type products can be made from a variety of materials including meltblown materials, coform materials, air-laid materials, bonded-carded web materials, hydroentangled materials, spunbond materials and similar materials and can include synthetic or natural fibers. Examples of natural fibers suitable for use in the present invention include cellulosic fibers such as wood pulp fibers, cotton fibers, flax fibers, jute fibers, silk fibers and similar natural fibers. Examples of thermoplastic polymeric fibers suitable for use with the present invention include polyolefins such as polypropylene and polyethylene, polyamides, and polyesters such as polyethylene teraphthalate. Alternative synthetic fibers which may be suitable include staple nylon and rayon fibers. The layer or layers of the wet wipe or wipe-type products can be woven or nonwoven materials.

If a layer of the basesheet is a combination of polymeric and natural fibers, such as polypropylene and cellulosic fibers, the relative percentages of the polymeric fibers and natural fibers in the layer can vary over a wide range depending on the desired characteristics of the wet wipes. For example, the layer may include from about 20 to about 95 weight percent, desirably from about 20 to about 68 weight percent, and more desirably from about 30 to about 50 weight percent of polymeric fibers based on the dry weight of the layer. Such a layer of polymeric and natural fibers may be manufactured by any method known to those skilled in the art.

Generally, it is desirable that such a layer be formed by a coform process for a more uniform distribution of the polymeric and natural fibers within the layer. Such coform layers are manufactured generally as described in U.S. Patent No. 4,100,324 to Anderson et al. which issued July 11, 1978; U.S. Patent No. 4,604,313 to McFarland et al. which issued August 5, 1986; and U.S. Patent No. 5,350,624 which issued September 27, 1994;

Typically, such coform layers include a gas-formed matrix of thermoplastic polymeric meltblown microfibers, such as, for example, polypropylene microfibers, and cellulosic fibers, such as, for example, wood pulp fibers. A coform layer is formed by initially forming at least one primary air stream containing the synthetic or polymeric fibers and merging the primary stream with at least one secondary stream of natural or cellulosic fibers. The primary and secondary streams are merged under turbulent conditions to form an integrated stream containing a thorough, homogeneous distribution of the different fibers. The integrated air stream is directed onto a forming surface to air form the layer of material. A multiplicity of these coform layers can then be formed in succession to provide a web of multiple coform layers.

The different fibers in the different layers of the layered basesheet of the present invention, such as the polypropylene and polyethylene microfibers set forth above, typically may not be compatible with and may not bond to each other. However, the different fibers may entangle with each other resulting in suitable securement between the layers. For example, in a layered basesheet containing a coform layer of polyethylene and cellulosic fibers and a coform layer of polypropylene and cellulosic fibers, the polyethylene and polypropylene fibers may entangle with each other and the cellulosic fibers and may at least partially bond to the cellulosic fibers which results in securement between the layers.

Such interlayer bonding and entanglement may be enhanced by a thermomechanical process wherein the layered basesheet is passed between a heated smooth anvil roll and a heated pattern roll. The pattern roll may have any raised pattern that provides the desired entanglement and interlayer bonding. Desirably, the pattern roll defines a raised pattern that defines a plurality of bond locations which define a bond area of between about 4 and about 30 percent of the total area of the roll for improved interlayer attachment.

The basesheet for the wet wipes or wipe-type products may have a total basis weight of from about 25 to about 120 grams per square meter and desirably from about 40 to about 90 grams per square meter. Such basis weight of the layered basesheet may also vary depending upon the desired end use of the wet wipe or wipe-type products. For example, a suitable basesheet for wiping the skin may define a basis weight of from about 60 to about 85 grams per square meter and desirably about 75 grams per square meter. In a particular embodiment wherein the basesheet includes coform layers of polypropylene and cellulosic fibers and polyethylene and cellulosic fibers, the layered basesheet defines a basis weight of from about 60 to about 90 grams per square meter and desirably about 85 grams per square meter for improved softness and adequate strength.

In a particular embodiment, it is desired that the wet wipe of the system of the present invention define sufficient strength to withstand the forces exerted by the user when it is wetted with solution. For example, the basesheet for the wet wipes or wipe-type products may define a tensile strength of at least about 1.23 Newtons per centimeter in the machine direction and at least about 0.70 Newtons per centimeter in the cross machine direction. The wipes or wipe-type having alternate ranges of tensile strength may also be effectively employed. As used herein, the term "machine direction" refers to the direction in which the material is manufactured while the cross machine direction refers to a direction that is perpendicular to the machine direction.

In a particular embodiment, wherein the basesheet includes coform layers of polypropylene and cellulosic fibers and polyethylene and cellulosic fibers, the layered basesheet defines a tensile strength of from about 1.31 to about 3.50 Newtons per centimeter in the machine direction and from about 0.84 to about 1.40 Newtons per centimeter in the cross machine direction and desirably from about 1.58 to about 1.93 Newtons per centimeter in the machine direction and from about 0.93 to about 1.11 Newtons per centimeter in the cross machine direction. In such a configuration, the coform layer that includes polypropylene fibers provides the majority of the strength to the basesheet while the coform layer that includes the polyethylene fibers provides a soft surface for contact with the skin of the user. Thus, the tensile strength of such a layered basesheet is higher than the tensile strength of a single layer containing polyethylene fibers and provides a softer surface than a single layer containing polypropylene fibers.

The wet wipes of the systems of the invention include a skin care solution. The skin care solutions of the invention can include from about 70 to about 99 percent by weight of one or more hydrophilic solvents. Desirably, the hydrophilic solvent is water. The water contained in the solution may be a mixture of water and alcohol. The preferred alcohols are ethanol and isopropyl alcohol. The amount of alcohol in the water is up to about 70 weight percent of the water and alcohol solution. More preferably, the amount of alcohol in the water is from about 40 to about 60 weight percent of the water and alcohol solution.

The skin care solutions of the invention can also include from about 0 to about 30 percent by weight of one or more surfactants. More specifically, the skin care solution can include from about 0.5 to about 20 percent by weight of surfactant. Desirably, the skin care solution can include from about 1 to about 15 percent by weight of surfactant. The surfactants of the skin care solution can include anionic, nonionic, cationic, zwitterionic, amphoteric and polymeric surfactants and mixtures thereof. One who is skilled in the art will recognize the functions and use of each of these classes of surfactants. Some botanicals and other skin care health benefiting agents are lipophilic and would require emulsification into the skin care solution. In order to emulsify lipophilic ingredients it may be necessary to use surfactants having an HLB of 6 to about 18 to stablize these lipophilic ingredient in the skin care solution.

Desirably, the surfactant of the skin care solution is selected from emulsifying wax NF, glyceryl stearate, glyceryl stearate SE glycol stearate, glycol stearate SE, glycereth-20 stearate, glyceryl behenate, glyceryl hydroxystearate, glyceryl laurate SE, glyceryl oleate, glyceryl oleate SE, propylene glycol oleate, propylene glycol oleate SE, propylene glycol stearate, propylene glycol stearate SE, sorbitan stearate, sorbitan trioleate acrylates/$C_{10-30}$ alkyl acrylate cross-polymer, dimethicone copolyol, sodium cocoamphoacetate, disodium lauroamphodiacetate, disodium caprylamphodipropionate, cocamidopropyl betaine, cluramidopropyl betaine, octyl betaine, cocamidopropyl hydroxysultaine, diammonium lauryl sulfsuccinate, disodium dimethicone copolyol sulfosuccinate, diammonium laureth sulfosuccinate, sodium lauryl sulfate, sodium laureth sulfate, ammoinum lauryl sulfate, TEA lauryl sulfate, polyoxyethylene hydrogenated castor oil, dimethicone copolyol sulfosuccinate, disodium cocoamphodiacetate, disodium isostearylamphoacetate, isostearylamidopropyl betaine, polysorbate 20, potassium laureth phosphate, sodium cocoamphoacetate, and sodium coco PG-dimonium chloride phosphate and mixtures thereof.

The skin care solutions also include extracted botanical actives. The extracted botanical actives of the skin care solutions are extracts, containing the chemically "active" components, of various plants and plant substances. Extracted botanical actives can include any water-soluble or oil-soluble active extracted from a particular plant. In addition, the botanical extracted actives can be supplied as a powder. The extracted botanical actives are actives extracted from echinacea, yucca, willow herb, black tea, Chinese tea, green tea (including epicatechin gallate and epigallocatechin 3-O-gallate), oolong tea, and any combinations thereof. Particular benefits have been observed with skin care solutions including echinacea, yucca glauca, green tea, black tea, oolong tea, Chinese tea, constituents of tea extracts and willow herb. Echinacea actives may be obtained from the following echinacea species: *Echinacea angustifolia, Echinacea purpurea,* and *Echinacea pallida.* Varieties of black tea include Flowery Orange Pekoe, Golden Flowery Orange Pekoe and Fine Tippy Golden Flowery Orange Pekoe. Varieties of green tea include Japanese and Green Darjeeling.

The skin care solutions of the invention include from about 0.1 to about 10 percent by weight of one or more extracted botanical actives. More specifically, the skin care solutions can include from about 0.5 to about 8 percent by weight of one or more extracted botanical actives. Even more specifically, the skin care solutions include from about 1 to about 5 percent by weight of extracted botanical actives. In particular aspects, the extracted botanical actives can be at least a minimum of about 0.1 percent by weight. The extracted botanical actives can alternatively be at least about 0.5 percent, and optionally, can be at least about 1 percent to provide improved performance. In other aspects, the extracted botanical actives can be not more than a maximum of about 10 percent by weight. The extracted botanical actives can alternatively be not more than about 8 percent, and optionally, can be not more than about 5 percent. Botanicals are primarily extracts of the plants from which they originate and botanicals are available from suppliers as part of a composition that may also contain an extracting solvent. Amounts of the botanicals in the skin care solutions of the invention in terms of active component (not extract) may range from about 0.000001 to about 10% by weight. Desirably, the amount of active botanical is from about 0.00001 to about 5% and more desirably from about 0.0001 to about 1 % by weight of the skin care solution. Further, it is also desirable that the amount of active botanical is from about 0.0001 to about 0.5% of the skin care solution and more desirably from about 0.001 to about 0.1 % by weight of the skin care solution.

The skin care solution incorporated into the wet wipe of the skin health improving system of the invention can include components in addition to the hydrophilic solvent, surfactant and extracted botanical active components. For example, the skin care solution can include an oil in water emulsion formed by incorporation of from about 0.1 to about 30 percent by weight of natural fats or oils with the hydrophilic solvent. More specifically, from about 0.5 to about 10 percent by weight of natural fats or oils can be used to transform the skin care solution into an emulsion. Even more specifically, from about 1 to about 5 percent by weight of natural fats or oils can be used to form an emulsion with the hydrophilic solvent of the skin care solution. Suitable natural fats or oils can be selected from avocado oil, apricot oil, babassu oil, borage oil, camellia oil, canola oil, castor oil, coconut oil, corn oil, cottonseed oil, evening primrose oil, hydrogenated cottonseed oil, hydrogenated palm kernel oil, maleated soybean oil, meadowfoam oil, palm kernel oil, phospholipids, rapeseed oil, palmitic acid, stearic acid, linoleic acid, stearyl alcohol, lauryl alcohol, myristyl alcohol, benenyl alcohol, rose hip oil, sunflower oil, soybean oil and mixtures of such natural fats and oils.

The skin care solutions of the invention can also incorporate sterols and sterol derivatives in such a way that an emulsion is formed with the hydrophilic solvent. For example, the skin care solution can include from about 0.1 to about 10 percent by weight of one or more sterols or sterol derivatives. More specifically, the skin care solution can include from about 0.5 to about 5 percent by weight of sterols or sterol derivatives. Even more specifically, the skin care solution can include from about 0.8 to about 3 percent by weight of sterols or sterol derivatives where the sterols and sterol derivatives form an emulsion with the hydrophilic solvent and other components. Suitable sterols and sterol derivatives can be selected from cholesterol, sitosterol, stigmasterol, ergosterol, lanasterol, soy sterol, avocado sterols, cholesterol esters, sterol esters, lanolin and mixtures thereof.

The skin care solutions of the invention can also include a humectant component. Humectants can be included in order to increase the water content of the top layers of the skin. Humectant materials include primarily hydroscopic ingredients. The skin care solutions of the invention can include from about 0.1 to about 30 percent by weight of one or more humectants. More specifically, the skin care solutions can include from about 0.5 to about 20 percent by weight of one or more humectants. Even more specifically, the skin care solutions can include from about 1 to about 10 percent by weight of one or more humectants. Suitable humectants may be selected from acetamide MEA, aloe barbadensis gel, arginine PCA, chitosan PCA, copper PCA, corn glycerides, dimethyl imidazolidinone, fructose, glucamine, glucose, glucose glutamate, glucuronic acid, glutamic acid, glycereth-7, glycereth-12, glycereth-20, glycereth-26, glycerin, honey, hydrogenated honey, hydrogenated starch hydrolysate, hydrolyzed corn starch, lactamide MEA, lactic acid, lactose lysine PCA, mannitol, methyl gluceth-10, methyl gluceth-20, PCA, PEG-2 lactamide, PEG-10 propylene glycol, polyamino sugar condensate, potassium PCA, propylene glycol, propylene glycol citrate, saccharide hydrolysate, saccharide isomerate, sodium aspartate, sodium lactate, sodium PCA, sorbitol, TEA-lactate, TEA-PCA, urea, xylitol and mixtures of such compounds.

The amount of skin care solution contained within each wet wipe may vary depending upon the type of material being used to provide the wet wipe or wipe-type product, the type of container being used to store the wet wipes, and the desired end use of the wet wipe. Generally, each wet wipe or wipe-type product can contain from about 100 to about 600 weight percent and desirably from about 250 to about 450 weight percent liquid based on the dry weight of the wipe for improved wiping. In a particular aspect, the amount of liquid contained within the wet wipe is from about 300 to about 400 weight percent and desirably about 330 weight percent based on the dry weight of the wet wipe. If the amount of liquid is less than the above-identified ranges, the wet wipe may be too dry and may not adequately perform. If the amount of liquid is greater than the above-identified ranges, the wet wipe may be oversaturated and soggy and the liquid may pool in the bottom of the container.

The hydrophilic skin care solution of the wet wipe is a good vehicle for delivering the extracted botanical actives to the skin. The extracted botanical actives have be shown to have good ability to reduce the inflammation response caused by irritants- particularly when delivered in a hydrophilic solution such as can be provided with a wet wipe.

In order to evaluate the efficacy of the skin care solutions of the invention, a human skin culture was selected to model the response of the human epidermis. EPIDERM skin culture is a cornified, air-interfaced human skin culture. EPIDERM skin culture has multiple layers of progressively differentiated keratinocytes resembling human epidermis. EPIDERM EPI-200 skin culture can be purchased from MatTek Corporation of Ashland, MA. Experiments using EPIDERM skin culture are conducted in six well plates. Each well contains one milliliter of pre-warmed media that is the same as the EPIDERM skin culture media. The plates are then incubated in a 37°C, 5% $CO_2$ incubator for thirty minutes. After incubation, 15 microliters of test composition or control are applied to the surface of the EPIDERM skin culture after removing any residual media. The well plates, with the test compositions/control applied, are incubated in the 37°C, 5% $CO_2$ incubator for thirty minutes after which the underlying media is removed and replaced with fresh, pre-warmed media. Next, ten microliters of a protease insult solution is applied to the surface of the EPIDERM skin culture.

Infant feces contain proteases that include trypsin and chymotrypsin (See Haverback, B. J., Dyce, B.J., Gutentag, P.J., and Montgomery, D. W. (1963) Measurement of Trypsin and Chymotrypsin in Stool. Gastroenterology 44: 588-597; and Barbero, G.J., Sibinga, M.S., Marino, J. M., and Seibel, R. (1966) Stool Trypsin and Chymotrypsin. Amer. J. Dis. Child 112:536-540). For internal studies, infant feces were collected and the amount of total protease and trypsin activities determined for each of the fecal extracts. To prepare the extract, the feces were suspended in water and vigorously vortexed. After vortexing, the samples were held on ice prior to centrifugation at 15,000 times the force of gravity for 20 minutes. The supernatant was filtered through 0.22 micron cellulose acetate filters and stored at -80°C until use. The amount of trypsin activity in the fecal extracts ranged from 0.4-402 $\mu$g/ml (n=19) as measured by the ability of the sample to hydrolyze a fluorescently-labeled trypsin peptide substrate (Boc-Gln-Ala-Arg-AMC HCl, BACHEM California, Incorporated, Torrance, CA). Total protease activity was measured as the ability of the sample to hydrolyze a fluorescent dye-labeled casein substrate (EnzChek Protease Assay Kit (E-6639), Molecular Probes, Eugene, OR). Irritation induced in the EPIDERM skin culture correlated with the total protease as well as trypsin activities of the fecal extracts. Based on the literature sources as well as internal data, a trypsin-chymotrypsin insult was chosen as representative of a fecal insult, specifically a fecal protease insult. See data in Table 1.

The insult solution used to generate data shown in Table 1 is prepared by diluting a 10 mg/ml stock solution in phosphate-buffered saline to a working concentration of 250 $\mu$g/ml. The base of the stock solution is 50 mM NaOAcetate, pH 5.5 and 0.15 M NaCl stored at -80°C. One milliliter of the stock protease insult solution contains 2558 USP units of trypsin and 298 USP units of chymotrypsin and is available from Specialty Enzymes, Inc. of Chino, CA. Phosphate-buffered saline, pH 7.4 (hereinafter "PBS") can be purchased from Life Technologies of Rockville, Maryland.

After application of the insult solution, the well plates are incubated for six hours in the 37°C, 5% $CO_2$ incubator. At the end of six hours, the well plates are removed from the incubator, the underlying media is removed and stored at -80°C. The response of the EPIDERM skin culture to the test compositions/control and the insult solution is determined by measuring the amount of interleukin-1 alpha (hereinafter "IL-1 $\alpha$"). Interleukin-1 alpha can be quantified using an Interleukin-1 alpha Quantikine Kit available from R&D Systems of Minneapolis, Minnesota. Interleukin-1 alpha measurements are converted to $Log_{10}$ for each of the treatments and the averages for each treatment are calculated. In order to determine the ability of the test compositions to reduce skin irritation caused by the biological insult, the percent mean reduction of IL-1$\alpha$ is calculated as follows:

$$\% \text{ mean reduction of IL-1}\alpha = 100 \times \frac{((\text{control} + \text{insult}) \text{ result} - (\text{test composition} + \text{insult}) \text{ result})}{((\text{control} + \text{insult}) \text{ result} - (\text{control} + \text{control}) \text{ result})}$$

(Test composition + insult) result = the measured amount of IL-1α from treatment with a test composition + protease insult.

(control + control) result = the measured amount of IL-1α from a treatment with a control with control.

For data shown in Table 1, (control + control) result= the measured amount of IL-1$\alpha$ from a treatment with PBS + PBS. For data shown in Table 2, (control + control) result= the measured amount of IL-1$\alpha$ from a treatment with Milli-Q water + Milli-Q water.

The greater the % mean reduction of IL-1$\alpha$, the more effective a composition is at reducing irritation caused by the protease insult.

In order to insure that the test compositions/control do not affect the viability of the EPIDERM skin culture, a MTT assay is run. The MTT dye is taken up by the cells. The reduction of the dye as a result of cellular metabolism can be used to measure the cytotoxicity of the test compositions. In order to confirm viability, inserts of the EPIDERM skin culture that have already been subjected to the test compositions and biological insults are removed from their media and are washed consecutively through immersion in three different beakers of PBS. Fresh PBS is used for each test composition or control being evaluated. The PBS is discarded onto paper towel. The EPIDERM skin culture inserts are then patted onto paper towel and placed into the wells of a 24 well plate containing 300 microliters of pre-warmed media. After all of the EPIDERM skin culture inserts are washed, they are transferred to new 24 well plates containing 300 microliters of the MTT reagent. The MTT reagent is thiazolyl blue having the formula 3-[4,5-Dimethylthiazol-2-yl]-2,5-diphenyltetrzoliumbromide. The plates are incubated for 2-3 hours in a 37°C, 5% $CO_2$ incubator. After incubation, the EPIDERM skin culture inserts are transferred to 24 well plates and are immersed in 2 milliliters of MTT extraction buffer. The extraction buffer extracts the MTT reagent from the cells. The 24 well plates are parafilmed, covered and placed in ZIPLOCK bags to reduce evaporation of the extraction buffer. The covered plates are rocked overnight in the dark. Following overnight rocking, the liquid in the EPIDERM skin culture inserts is decanted back into the wells. The contents of each well are mixed and a 200 microliter aliquot is then removed from each well and transferred to a 96 well plate. The optical density (OD) of the samples is measured at 570 nm using a spectrophotometer. Five hundred seventy nanometers is the optimal wavelength at which to measure the reduced form of MTT reagent. This reading is subtracted from a background reading at 650 nm to improve data quality. Percent viability of each test composition + insult relative to a negative control + control is recorded as the Mean $OD_{\text{test composition+insult}}$ divided by the Mean OD control + control; the quotient then multiplied by 100.

EPIDERM skin culture studies were conducted to measure the reduction in IL-1$\alpha$ response between skin care solutions of the invention and a fecal protease-induced irritation. The studies were conducted using botanicals that can be delivered in the skin care solutions of the invention. The EPIDERM skin culture studies and associated MTT assays were conducted as already described herein and the results are as reported in Table 1. below.

**Table 1.**

| Botanical Component of Solution | Mean Reduction of Interleukin-1 Alpha (percentage) | Viability (percentage) |
|---|---|---|
| 1% Echinacea | 20% (5) ; 39% (10)* | 89% ; 90% |
| 10% Echinacea 1% Yucca | 22% (5) ; 31% (10)* | 80% ; 86% |
|  | 27% (5)* ; 54% (10)* | 84% ; 95% |
| 10% Yucca | 21% (5); 18% (10) | 83% ; 81 % |
| 1% Willow Herb | 8% (10); 38% (5) | 74% ; 98% |
| 10% Willow Herb | 81% (10)* ; 99% (4)* | 92% ; 103% |
| 0.4% Epigallocatechin gallate (component of green tea) | 77% (5)* ; 71% (10)* ; 50% (5) | 103% ; 101%; 112% |
| "*" indicates the composition had a significant mean difference from the protease insult applying a Student's t-test with p<0.05. | | |

The IL-1$\alpha$ reduction results of Table 1. show that the skin care solutions of the invention provide a skin protectant effect as evidenced by a reduced irritation response. At least one set of experiments were conducted for each botanical and, for some botanicals, more than one set of experiments was conducted. The values in parentheses indicate the number of replicates. All botanicals were received as solutions and diluted with PBS (v/v) to desired dilutions (1 and 10%) while epigallocatechin gallate was weighed out and dissolved in PBS to desired levels (w/v). The sources of the botanicals were as follows: Echinacea from Bio-Botanica; Yucca Glauca from Brooks; Willow Herb from Fytokem; and Epigallocatechin Gallate from Sigma Chemical Company.

The reduction of IL-1$\alpha$ results were analyzed to statistically indentify "outlier" results. The EPIDERM skin culture is known to be variable with the variability attributed to differences in the culture, variation in the application of treatment and other uncontrollable factors. A statistical analysis technique was applied to identify when a result abnormally deviated from the rest of the data set. The irritation values were first converted to Log10 in order to make them more Gaussian (bell curve-shaped). After conversion, the values were analyzed for high or low value outliers; subsequently, the values were analyzed with a student's t-test to identify significant differences from the "control". The statistical analysis used to identify "outliers" is described on page 460 of the book, "Statistical Methods in Research and Production" edited by Owen L. Davies and Peter L. Goldsmith, published by Longman Group Limited, fourth revised edition published in 1984.

The EPIDERM skin culture experiments described above measure the anti-irritant effect of botanicals contained in a buffered saline solution, PBS. EPIDERM skin culture experiments were also conducted to determine whether the extracted botanical active of the skin care solution retained its efficacy in a typical wet wipe solution. The experiments were designed to determine whether ingredients in the expressed wet wipe solution interfered with the skin health benefit (s) of the botanical. This set of experiments was conducted as described above, with a few exceptions. First, the underlying media is removed and replaced with 1 mL of fresh, prewarmed media after the initial 30 minute acclimation of the skin culture instead of after the 30 minute treatment. Second, a different insult solution was used. A 10 mg/mL stock solution of trypsin (15,200 U/mg protein, available from Sigma Chemical Company, St. Louis, MO) in Mili-Q water was prepared the day of the study and diluted to a working concentration of 250 $\mu$g/mL just prior to beginning the test. Third, the well plates are incubated up to 24 hours in a 37°C 5% CO2 incubator instead of six. Interleukin-1 alpha levels are quantified from 50 $\mu$l aliquots taken at 6 and 12 hours as well as the underlying media following the 24 hour incubation. HUGGIES SUPREME CARE wet wipes (fragrance-free with aloe) were obtained from Kimberly-Clark Corporation. The solution was expressed from the wet wipes by hand on the day of the test. Green tea extract (available from Dragoco, Totowa, NJ) was dissolved in either Mili-Q water or the expressed wet wipe solution to prepare a 0.5% (w/v) solution. The reduction in IL-1$\alpha$ was measured and the results are reported in Table 2. below.

**Table 2.**

| Test Solution | Mean Reduction Interleukin-1 Alpha- 6 hours (percentage) | Mean Reduction Interleukin-1 Alpha- 12 hours (percentage) | Mean Reduction Interleukin-1 Alpha- 24 hours (percentage) |
|---|---|---|---|
| Green tea extract in water (0.5%) | 33 (4) | 35 (4) | 29 (4) |

(continued)

| Test Solution | Mean Reduction Interleukin-1 Alpha- 6 hours (percentage) | Mean Reduction Interleukin-1 Alpha- 12 hours (percentage) | Mean Reduction Interleukin-1 Alpha- 24 hours (percentage) |
|---|---|---|---|
| Green tea extract in HUGGIES baby wipe expressed solution (0.5%) | 53 (6) | 40 (6) | 23 (6) |

The number of replicates for each solution is indicated in parentheses; six replicates were conducted for the test solution of green tea extract in HUGGIES SUPREME CARE expressed solution and four replicates were conducted for green tea extract in water (two culture inserts had - 40% loss of cell viability and were not included in the calculations.)The data show that the inclusion of a green tea extract reduces the mean IL-1$\alpha$ skin irritation score in a wet wipe expressed solution (HUGGIES SUPREME CARE (fragrance-free with aloe)) at comparable levels to water. Green tea extract, components derived from the tea extract, and other similar botanicals based on these data should remain effective at reducing fecal irritation, in particular irritation associated with protease-induced skin irritation, in aqueous wet wipe formulations.

In order to demonstrate the benefits of the systems of the invention, experiments were conducted to investigate the amount of skin care composition and skin care solution transferred to the skin when the absorbent articles and wet wipes of the invention are used together. The experiments were designed to investigate whether the sequence of application (diaper or wet wipe) affected the amounts of skin care composition/solution transferred and to investigate whether there is a benefit to using the diapers and wet wipes of the invention together or if the cancel out each other's effects.

## Transfer Test Method

A method was developed for measuring the amount of composition or solution transferred from a treated non-woven surface. Composition/solution transfer is measured gravimetrically using an Ink Rub Tester such as Model #10-18-01 manufactured by Testing Machines Inc. For purposes of the method described herein, the Ink Rub Tester is set to have the following settings: (1) Cycles = 50; (2) Cycles/minute = 42; (3) Alarm = on; (4) Pause every = off; and (5) Count = down. A four pound weighted block is used in order to more closely simulate the pressure applied by an infant to an absorbent article, such as a diaper, while sitting or moving or a wet-wipe when used to wipe infant skin. The receptor or receiving material is held to the four pound weighted block by four magnets. The four magnets are positioned so that there are two evenly spaced on either side of the four pound weighted block. Typically, the receptor material is Natural Silk Noil, style #651, which is available from Testfabrics, Inc. The receptor material is cut to have dimensions of 5.08 cm (2 inches) by 15.24 cm (6 inches). Desirably, the magnets are high powered neodymium magnets having a diameter of 0.64 cm (0.25 inches) and a height of 0.64 cm (0.25 inches). Preferably, the tension on the receptor material is uniform and the receptor material is held tightly with no gaps between the weighted block and the receptor material. The receptor material should not, however, be subjected to extraordinary strain.

The treated bodyside liner material or wet wipe substrate that is treated with a composition/solution is centered on a rubber pad that will be brought into contact with the four pound weighted block during the test. The bodyside liner material is typically a spunbond liner material and the wet-wipe is a typically a co-formed non-woven material. The planar dimension of the rubber pad is approximately 6.35 cm (2.5 inches) by 15.24 cm (6 inches). The liner material is usually cut to dimensions of approximately 7.62 cm (3 inches) by 22.86 cm (9 inches) in order to completely cover the rubber pad. In case of the wet-wipe it is typically folded to conform to the 7.62 cm (3 inches) by 22.86 cm (9 inches format). A composition is applied to the liner material using a slot-coating technique. Desirably, the slot-coated lines of composition are aligned lengthwise with the length of the rubber pad and the lines are centered as much as possible. The solution for the wet-wipe is adsorbed onto the co-form base-sheet. Both ends of the rubber pad have strips of hook 88 VELCRO tape adhered to them. The liner or wet-Wipe material is held in place on the rubber pad by pressing the liner material against the VELCRO tapes. The liner or wet-wipe material should be as wrinkle-free as possible when positioned against the rubber pad. Two, three inch (7.62 cm) wide IDL ball bearing clips are then attached to either end of the rubber pad in order to hold the liner or wet-wipe material in place and to prevent movement of the liner or wet-wipe material.

Prior to attachment to the four pound weighted block, the receptor material should be weighed on a balance that is significant to at least four decimal places. Prior to placement on the weighing plate of the balance, the receptor material should be tri-folded so that no portion is hanging off of the weighing plate. Care should be taken to make sure there are no threads hanging from the receptor material as well. The initial mass of the receptor material is recorded.

Gloves should be worn during handling of each of the materials in order to prevent transfer of oils naturally present on hands to the test materials. Once both the receptor material and liner material are secured, the test is conducted using the Ink Rub Tester settings described previously. At the end of the test, the liner material is removed and thrown away. The receptor material is removed and sprayed with Milty Zerostat 3 Anti-Static Pistol available from SPI Supplies. After removal of static, the receptor material is once again tri-folded and its mass is recorded using the balance. The change in mass is the difference between the initial and final masses of the receptor material and is representative of the amount of composition transferred from the liner or wet-wipe material.

### Effect of Treated Liner Followed by Treated Wet-Wipe

An Ink Rub Tester test was conducted on "control" compositions including only petrolatum and a wax (60% petrolatum; 34% ozokerite wax; and 6% ELVAX 410 resin) on the diaper liner and a test solutions (solution from HUGGIES SUPREME CARE baby wipes) representative of wet-wipe solutions of the invention. The objective of the test was to determine the additive amount of skin treatments delivered to the receptor material when applying the diaper liner treatment immediately followed by the wet-wipe treatment. The measurements were conducted in two steps: 1) The amount of transfer from the treated diaper liner was first measured and 2) Using the same receptor material the treated wet-wipe was applied to the receptor and the amount transferred measured after allowing the receptor to air dry at room conditions for a period of 12 hours. The reason for the drying period was to allow the receptor to equilibrate after becoming wet after the wet wipe treatment. Immediate weighing after the wet-wipe treatment would have greatly exaggerated the amount of treatment actually transferred from the wet wipe. The measurements determined that the additive amount of treatment delivered by the combined use of the liner treatment and wet wipe treatment was greater than the amount of treatment delivered by either the liner or the wet wipe alone, thus illustrating the advantages of a system of the invention that includes the combined usage of a treated diaper and a treated wet-wipe. The additive effect of a liner treatment followed by a treated wet wipe was repeated in two separate transfer tests and the gravimetric transfer data are presented in Table 3. below.

**Table 3.**

| TEST #1 Repetition | ∆Wt. After Liner Trt. | ∆Wt. After Wet-Wipe | Total A Wt. After Liner + Wet-Wipe |
|---|---|---|---|
| 1 | 0.0071 | 0.0012 | 0.0083 |
| 2 | 0.0083 | 0.0073 | 0.0156 |
| 3 | 0.0082 | 0.0012 | 0.0094 |
| 4 | 0.0098 | 0.0030 | 0.0101 |
| 5 | 0.0103 | 0.0017 | 0.0120 |
| 6 | 0.0064 | -0.0036 | 0.0028 |
| 7 | 0.0038 | 0.0071 | 0.0109 |
| 8 | 0.0079 | 0.0022 | 0.0101 |
| 9 | 0.0075 | 0.0042 | 0.0117 |
| 10 | 0.0106 | -0.0010 | 0.0096 |
| **Avg.** | **0.0080** | **0.0021** | **0.0101** |
| **TEST #2 Repetition** | **∆Wt. After Liner Trt.** | **∆Wt. After Wet-Wipe** | **Total A Wt. After Liner + Wet-Wipe** |
| 11 | 0.0012 | 0.0096 | 0.0108 |
| 12 | 0.0021 | 0.0135 | 0.0156 |
| 13 | 0.0100 | 0.0220 | 0.0320 |
| 14 | 0.0130 | 0.0017 | 0.0147 |
| 15 | 0.0039 | 0.0088 | 0.0127 |
| 16 | 0.0037 | 0.0195 | 0.0232 |
| 17 | 0.0078 | 0.0086 | 0.0164 |
| 18 | 0.0030 | 0.0106 | 0.0136 |
| 19 | 0.0149 | -0.008 | 0.0069 |
| 20 | 0.0023 | 0.0103 | 0.0126 |

(continued)

| TEST #2 | | | |
|---|---|---|---|
| Repetition | ΔWt. After Liner Trt. | ΔWt. After Wet-Wipe | Total A Wt. After Liner + Wet-Wipe |
| Avg. | 0.0062 | 0.0966 | 0.1028 |
| (Note: All measurements are in grams weight) | | | |

## Effect of Treated Wet-Wipe Followed by Treated Liner

An additional Ink Rub Tester test was conducted on representative skin care compositions including only petrolatum and a wax on the diaper liner and solutions representative of skin care solutions of the invention. In this test, the order of application was reversed, in that the treated wet wipe was transferred to the receptor material, allowed to dry for 12 hours and then to the same receptor the treated liner was transferred. The objective of the test was to determine the additive amount of skin treatment delivered to the receptor material when applying the treated wet wipe followed by the treated liner. The measurements were conducted in two steps: 1) The amount of transfer from the wet wipe was first measured after allowing the receptor to air dry at room conditions for a period of 12 hours and 2) Using the same receptor material the treated liner was applied to the receptor and the amount transferred measured. Again, the reason for the drying period was to allow the receptor to equilibrate after becoming wet after the wet wipe treatment. Immediate weighing after the wet wipe treatment would have greatly exaggerated the amount of treatment actually transferred from the wet wipe. The measurements determined that the additive amount of treatment delivered by the combined use of the treated wet wipe treatment followed by the treated liner was greater than the amount of treatment delivered by either liner or wet wipe alone, thus illustrating the advantage of a skin health improving system based on the combined usage of a treated wet wipe and a treated liner. The additive effect of a treated wet wipe followed by a treated liner was repeated in two separate transfer tests and the gravimetric transfer data are presented in Table 4. below.

**Table 4.**

| TEST #1 | | | |
|---|---|---|---|
| Repetition | ΔWt. After Wet Wipe. | ΔWt. After Treated Liner | Total Δ Wt. After Wet-Wipe + Liner |
| 21 | 0.0108 | 0.0150 | 0.0258 |
| 22 | 0.0111 | 0.0052 | 0.0163 |
| 23 | 0.0091 | 0.0108 | 0.0199 |
| 24 | 0.0098 | 0.0074 | 0.0172 |
| 25 | 0.0069 | 0.0048 | 0.0117 |
| 26 | 0.0116 | 0.0041 | 0.0157 |
| 27 | 0.0094 | 0.0063 | 0.0157 |
| 28 | 0.0149 | 0.0066 | 0.0215 |
| 29 | 0.0098 | 0.0039 | 0.0137 |
| 30 | 0.0105 | 0.0081 | 0.0186 |
| Avg. | 0.0104 | 0.0072 | 0.0176 |

| TEST #2 | | | |
|---|---|---|---|
| Repetition | ΔWt. After Liner Trt. | ΔWt. After Wet-Wipe | Total Δ Wt. After Liner + Wet-Wipe |
| 31 | 0.0117 | 0.0138 | 0.0255 |
| 32 | 0.0143 | 0.0034 | 0.0177 |
| 33 | 0.0110 | 0.0130 | 0.0240 |
| 34 | 0.0136 | 0.0075 | 0.0211 |
| 35 | 0.0103 | 0.0018 | 0.0121 |
| 36 | 0.0128 | 0.0134 | 0.0262 |
| 37 | 0.0133 | 0.0041 | 0.0174 |
| 38 | 0.0160 | 0.0032 | 0.0192 |
| 39 | 0.0113 | 0.0123 | 0.0236 |
| 40 | 0.0203 | 0.0108 | 0.0311 |

(continued)

| TEST #2 | | | |
| --- | --- | --- | --- |
| Repetition | ΔWt. After Liner Trt. | ΔWt. After Wet-Wipe | Total Δ Wt. After Liner + Wet-Wipe |
| Avg. | 0.0135 | 0.0083 | 0.0218 |
| (Note: All measurements are in grams weight) | | | |

The systems of the invention are configured to provide non-aqueous skin care compositions from an absorbent article (such as a disposable diaper) and hydrophilic skin care solutions from a wet wipe to work synergistically to provide improved skin health to wearers of absorbent articles. Skin care excipients having non-aqueous properties can be delivered in the skin care composition of the absorbent article and skin care excipients (such as extracted botanical actives) having better efficacy in a hydrophilic system can be delivered in the skin care solution of the wet wipe. The results of the experiments described herein show that extracted botanical actives can provide a skin protectant effect when delivered from a wet wipe solution. Separate experimental results show that the absorbent articles and wet wipes of the invention can be used together to deliver skin care treatments without interfering with each other. While it is believed that greater improvements in skin health can be achieved with more frequent usage of the systems of the invention, benefits can still be achieved with only sporadic usage as well.

While the invention has been described in detail with respect to the specific aspects thereof, it will be appreciated that those skilled in the art, upon attaining an understanding of the foregoing, may readily conceive of alterations to, variations of, and equivalents to these aspects. Accordingly, the scope of the present invention should be assessed as that of the appended claims and any equivalents thereto.

## Claims

1.   A system for improving skin health of a wearer of absorbent articles comprising:

   (a) a disposable absorbent article that includes an outer cover; a liquid permeable bodyside liner that defines a bodyfacing surface and that is connected in superposed relation to the outer cover; an absorbent body that is located between the bodyside liner and the outer cover; and a skin care composition on at least a portion of the bodyfacing surface of the bodyside liner that includes from 40 to 95 percent by weight of emollient and from 5 to 60 percent by weight of viscosity enhancer; and
   (b) a wet wipe that includes a nonwoven substrate and a skin care solution that includes from 90 to 99 percent by weight of hydrophilic solvent, from 0 to 30 percent by weight of surfactant and from 0.1 to 10 percent by weight of extracted botanical active selected from actives extracted from echinacea, yucca, willow herb, green tea, black tea, oolong tea, Chinese tea, constituents of tea extract and mixtures thereof.

2.   The system of claim 1, wherein the extracted botanical active is epicatechin gallate or epigallocatechin 3-O-gallate.

3.   The system of claim 1 or claim 2, wherein the emollient of the skin care composition is selected from petrolatum, vegetable based oils, mineral oils, dimethicone, lanolin, glycerol esters, alkoxylated carboxylic acids, alkoxylated alcohols, fatty alcohols and mixtures thereof.

4.   The system of any preceding claim, wherein the viscosity enhancer of the skin care composition is selected from polyolefin resins, lipophilic/oil thickeners, ethylene/vinyl acetate copolymers, natural clays, synthetic analogs of natural clays, organically modified clays, quaternary modified clays, quaternary starch compounds, polyethylene, silica, silica silylate, silica methyl silylate, colloidal silicone dioxide, alkyl hydroxy ethyl cellulose, microcrystalline wax, shellac wax, hexadecyl cosanyl hexacosanate, $C_{20}$-$C_{40}$ alkyl hydroxystearyl stearate, glycol montanate, ozokerite wax, polyperfluoromethylisopropylether montan wax, magnesium aluminum silicate, polymethacrylate polymers, polystyrene copolymers and mixtures thereof.

5.   The system of any preceding claim, wherein the skin care composition further includes from 5 to 55 percent by weight of solidifying agent.

6.   The system of claim 5 wherein the solidifying agent of the skin care composition is selected from beeswax, behenyl behenate, behenyl benzoate, branched esters, candelilla wax, carnauba wax, synthetic carnauba wax, PEG -12 carnauba wax, cerasin, microcrystalline wax, hydrogenated microcrystalline wax, hexadecylcosanyl hexacosanate, polyperfluoromethylisopropylether montan wax, alkylmethylsiloxanes, glycol montanate, jojoba wax, lanolin wax, ozokerite, paraffin, synthetic paraffin, polyethylene, $C_{20}$-$C_{40}$ alkyl hydroxystearyl stearate, $C_{30}$ alkyl dimethicone, cetyl esters, zinc stearate, shellac wax, hydrogenated cottonseed oil, hydrogenated squalene, hydrogenated jojoba oil and mixtures thereof.

**7.** The system of any preceding claim, wherein the skin care composition further includes from 0.1 to 55 percent by weight of natural fats or oils.

**8.** The system of claim 7, wherein the natural fat or oil is selected from Avocado Oil, Apricot Oil, Babassu Oil, Borage Oil, Camellia Oil, Canola Oil, Castor Oil, Coconut Oil, Corn Oil, Cottonseed Oil, Evening Primrose Oil, Hydrogenated Cottonseed Oil, Hydrogenated Palm Kernel Oil, Maleated Soybean Oil, Meadowfoam Oil, Palm Kernel Oil, Peanut Oil, Rapeseed Oil, Safflower Oil, Sphingolipids, Sweet Almond Oil, Tall Oil, Lauric Acid, Palmitic Acid, Stearic Acid, Linoleic Acid, Stearyl Alcohol, Lauryl Alcohol, Myristyl Alcohol, Behenyl Alcohol, Rose Hip Oil, Calendula Oil, Chamomile Oil, Eucalyptus Oil, Juniper Oil, Sandlewood Oil, Tea Tree Oil, Sunflower Oil, Soybean Oil and mixtures thereof.

**9.** The system of any preceding claim, wherein the skin care composition further includes from 0.1 to 10 percent by weight of sterols or sterol derivatives.

**10.** The system of claim 9, wherein the sterol or sterol derivative is selected from cholesterol, sitosterol, stigmasterol, and ergosterol, as well as, $C_{10}$-$C_{30}$ cholesterol/lanosterol esters, cholecalciferol, cholesteryl hydroxystearate, cholesteryl isostearate, cholesteryl stearate, 7-dehydrocholesterol, dihydrocholesterol, dihydrocholesteryl octyldecanoate, dihydrolanosterol, dihydrolanosteryl octyldecanoate, ergocalciferol, tall oil sterol, soy sterol acetate, lanasterol, soy sterol, avocado sterols, sterol esters and mixtures thereof.

**11.** The system of any preceding claim, wherein the surfactant of the skin care solution has a HLB range of from 6 to about 18.

**12.** The system of claim 11, wherein the surfactant of the skin care solution is selected from emulsifying wax NF, glyceryl stearate, glyceryl stearate SE glycol stearate, glycol stearate SE, glycereth-20 Stearate, glyceryl behenate, glyceryl hydroxystearate, glyceryl laurate SE, glyceryl oleate, glyceryl oleate SE, propylene glycol oleate, propylene glycol oleate SE, propylene glycol stearate, propylene glycol stearate SE, sorbitan stearate, sorbitan trioleate, dimethicone copolyol, sodium cocoamphoacetate, disodium lauroamphodiacetate, disodium caprylamphodipropionate, cocamidopropyl betaine, cluramidopropyl betaine, octyl betaine, cocamidopropyl hydroxysultaine, diammonium lauryl sulfsuccinate, disodium dimethicone copolyol sulfosuccinate, diammonium laureth sulfosuccinate, sodium lauryl sulfate, sodium laureth sulfate, ammoinum lauryl sulfate, TEA lauryl sulfate, polyoxyethylene hydrogenated castor oil and mixtures thereof.

**13.** The system of any preceding claim, wherein the skin care solution further includes from 0.1 to 30 percent by weight of natural fats or oils.

**14.** The system of claim 13, wherein the natural fats or oils are selected from avocado oil, apricot oil, babassu oil, borage oil, camellia oil, canola oil, castor oil, coconut oil, com oil, cottonseed oil, evening primrose oil, hydrogenated cottonseed oil, hydrogenated palm kernel oil, maleated soybean oil, meadowfoam oil, palm kernel oil, phospholipids, rapeseed oil, palmitic acid, stearic acid, linoleic acid, stearyl alcohol, lauryl alcohol, myristyl alcohol, benenyl alcohol, rose hip oil, sunflower oil, soybean oil and mixtures thereof.

**15.** The system of any preceding claim, wherein the skin care solution further includes from 0.1 to 10 percent by weight of sterol or sterol derivative.

**16.** The system of claim 15, wherein the sterol or sterol derivative is selected from cholesterol, sitosterol, stigmasterol, ergosterol, lanasterol, soy sterol, avocado sterols, cholesterol esters, sterol esters, lanolin and mixtures thereof.

**17.** The system of any preceding claim, wherein the skin care solution further includes from 0.1 to 30 percent by weight of humectant.

**18.** The system of claim 17, wherein the humectant is selected from acetamide MEA, aloe vera gel, arginine PCA, chitosan PCA, copper PCA, com glycerides, dimethyl imidazolidinone, fructose, glucamine, glucose, glucose glutamate, glucuronic acid, glutamic acid, glycereth-7, glycereth-12, glycereth-20, glycereth-26, glycerin, honey, hydrogenated honey, hydrogenated starch hydrolysate, hydrolyzed com starch, lactamide MEA, lactic acid, lactose lysine PCA, mannitol, methyl gluceth-10, methyl gluceth-20, PCA, PEG-2 lactamide, PEG-10 propylene glycol, polyamino sugar condensate, potassium PCA, propylene glycol, propylene glycol citrate, saccharide hydrolysate, saccharide isomerate, sodium aspartate, sodium lactate, sodium PCA, sorbitol, TEA-lactate, TEA-PCA, urea, xylitol and mixtures thereof.

## Patentansprüche

1. System zur Verbesserung der Hautgesundheit eines Trägers von absorbierenden Gegenständen, umfassend:

(a) Einen absorbierenden Wegwerfgegenstand, der eine äußere Abdeckung; eine flüssigkeitsdurchlässige, körperseitige Einlage, die eine zum Körper weisende Oberfläche definiert und in übereinandergelegter Beziehung mit der äußeren Abdeckung verbunden ist; einen absorbierenden Körper, der zwischen der körperseitigen Einlage und der äußeren Abdeckung angeordnet ist; und eine Hautpflegezusammensetzung auf mindestens einem Teil der zum Körper weisenden Oberfläche der körperseitigen Einlage, die von 40 bis 95 Gew.-% Erweichungsmittel und von 5 bis 60 Gew.-% eines Mittels zu Erhöhung der Viskosität enthält, einschließt; und
(b) ein feuchtes Wischtuch, welches ein Vliessubstrat und eine Hautpflegelösung umfasst, die von 90 bis 99 Gew.-% hydrophiles Lösungsmittel, von 0 bis 30 Gew.-% oberflächenaktives Mittel und von 0,1 bis 10 Gew.-% extrahierten pflanzlichen Wirkstoff enthält, ausgewählt aus Wirkstoffen, die aus Echinacea, Yucca, Weidenröschen, Grünem Tee, Schwarzem Tee, Oolongtee, chinesischem Tee, Bestandteilen von Teeextrakt und Gemsichen davon extrahiert sind.

2. System nach Anspruch 1, wobei der extrahierte pflanzliche Wirkstoff Epicatechingallat oder Epigallocatechin-3-O-gallat ist.

3. System nach Anspruch 1 oder 2, wobei das Erweichungsmittel der Hautpflegezusammensetzung aus Petrolatum, auf Pflanzen basierenden Ölen, Mineralölen, Dimethicon, Lanolin, Glycerinestern, alkoxylierten Carbonsäuren, alkoxylierten Alkoholen, Fettalkoholen und Mischen davon ausgewählt ist.

4. System nach einem vorhergehenden Anspruch, wobei das Mittel zur Erhöhung der Viskosität der Hautpflegezusammensetzung aus Polyolefinharzen, lipophilen/Öl-Verdickungsmitteln, Ethylen/Vinylacetat-Copolymeren, natürlichen Tonen, synthetischen Analogen von natürlichen Tonen, organisch modifizierten Tonen, quarternär modifizierten Tonen, quaternären Stärke-Verbindungen, Polyethylen, Siliciumdioxid, silyliertem Siliciumdioxid, methylsilyliertem Siliciumdioxid, kolloidalem Siliciumdioxid, Alkylhydroxyethylcellulose, mikrokristallinem Wachs, Schellackwachs, Hexadecylcosanylhexacosanat, $C_{20}$-$C_{40}$-Alkylhydroxystearylstearat, Glycolmontanat, Ozokeritwachs, Polyperfluormethylisopropylether-Montanwachs, Magnesiumaluminiumsilikat, Polymethacrylat-Polymeren, Polystyrol-Copolymeren und Mischungen davon ausgewählt ist.

5. System nach einem vorhergehenden Anspruch, wobei die Hautpflegezusammensetzung des Weiteren von 5 bis 55 Gew.-% verfestigendes Mittel enthält.

6. System nach Anspruch 5, wobei das verfestigende Mittel der Hautpflegezusammensetzung aus Bienenwachs, Behenylbehenat, Behenylbenzoat, verzweigten Estern, Candellilawachs, Carnaubawachs, synthetischem Carnaubawachs, PEG-12-Carnaubawachs, Cerasin, mikrokristallinem Wachs, hydriertem mikrokristallinem Wachs, Hexadecylcosanylhexacosanat, Polyperfluormethylisopropylether-Montanwachs, Alkylmethylsiloxanen, Glycolmontanat, Jojobawachs, Lanolinwachs, Ozokerit, Paraffin, synthetischem Paraffin, Polyethylen, $C_{20}$-$C_{40}$-Alkylhydroxystearylstearat, $C_{30}$-Alkyldimethicon, Cetylestern, Zinkstearat, Schellackwachs, hydriertem Baumwollsamenöl, hydriertem Squalen, hydriertem Jojobaöl und Mischungen davon ausgewählt ist.

7. System nach einem vorhergehenden Anspruch, wobei die Hautpflegezusammensetzung des Weiteren von 0,1 bis 55 Gew.-% natürliche Fette oder Öle enthält.

8. System nach Anspruch 7, wobei das natürliche Fett oder Öl aus Avocadoöl, Aprikosenöl, Babassuöl, Gurkenkrautöl, Kamelienöl, Rapsöl, Rizinusöl, Kokosnussöl, Maisöl, Baumwollsamenöl, Nachtkerzenöl, hydriertem Baumwollsamenöl, hydriertem Palmkernöl, maleiertem Sojabohnenöl, Sumpfblumenöl, Palmkernöl, Erdnussöl, Rapssamenöl, Färberdistelöl, Sphingolipiden, Süßmandelöl, Tallöl, Laurinsäure, Palmitisäure, Stearinsäure, Linolensäure, Stearylalkohol, Laurylalkohol, Myristylalkohol, Behenylalkohol, Hagebuttenöl, Ringelblumenöl, Kamillenöl, Eukalyptusöl, Wacholderöl, Sandelholzöl, Teebaumöl, Sonnenblumenöl, Sojabohnenöl und Mischungen davon ausgewählt ist.

9. System nach einem vorhergehenden Anspruch, wobei die Hautpflegezusammensetzung des Weiteren von 0,1 bis 10 Gew.-% Sterole oder Sterol-Derivate enthält.

10. System nach Anspruch 9, wobei das Sterol oder Sterol-Derivat aus Cholesterin, Sitosterol, Stigmasterol und Ergosterol sowie $C_{10}$-$C_{30}$-Cholesterin/Lanosterolestern, Cholecalciferol, Cholesteryl-hydroxystearat, Cholesteryl-isostearat, Cholesterylstearat, 7-Dehydrocholesterin, Dihydrocholesterin, Dihydrocholesteryl-octyldecanoat, Dihydrolanosterol, Dihydrolanosteryl-octyldecanoat, Ergocalciferol, Tallölsterol, Sojasterolacetat, Lanasterol, Sojasterol, Avocadosterolen, Sterolestern, und Mischungen davon ausgewählt ist.

11. System nach einem vorhergehende Anspruch, wobei das oberflächenaktive Mittel der Hautpflegelösung einen HLB-Bereich von 6 bis etwa 18 aufweist.

12. System nach Anspruch 11, wobei das oberflächenaktive Mittel der Hautpflegelösung aus emulgiertem Wachs NF, Glycerylstearat, Glycerylstearat-SE-gylcolstearat, Glycolstearat SE, Glycereth-20-stearat, Glycerylbehenat, Glycerylhydroxystearat, Glyceryllaurat SE, Glyceryloleat, Glyceryloleat SE, Propylenglycololeat, Propylenglycololeat SE, Propylenglycolstearat, Propylenglycolstearat SE, Sorbitanstearat, Sorbitantrioleat, Dimethiconcopolyol, Natriumcocoamphoacetat, Dinatriumlauroamphodiacetat, Dinatriumcaprylamphodipropionat, Cocamidopropylbetain, Cluramidopropylbetain, Octylbetain, Cocamidopropylhydroxysultain, Diammoniumlaurylsulfosuccinat, Dinatriumdimethiconcopolyolsulfosuccinat, Diammoniumlaurethsulfosuccinat, Natriumlaurylsulfat, Natriumlaurethsulfat, Ammoniumlaurylsulfat, TEA-Laurylsulfat, Polyoxyethylen-hydriertem Rizinusöl und Mischungen davon ausgewählt ist.

13. System nach einem vorhergehenden Anspruch, wobei die Hautpflegelösung des Weiteren von 0,1 bis 30 Gew.-% natürliche Fette oder Öl enthält.

14. System nach Anspruch 13, wobei die natürlichen Fette oder Öle aus Avocadoöl, Aprikosenöl, Babassuöl, Gurkenkrautöl, Kamillenöl, Rapsöl, Rizinusöl, Kokosnussöl, Maisöl, Baumwollsamenöl, Nachtkerzenöl, hydriertem Baumwollsamenöl, hydriertem Palmkernöl, maleiertem Sojabohnenöl, Sumpfblumenöl, Palmkernöl, Phospholipiden, Rapssamenöl, Palmitinsäure, Stearinsäure, Linolensäure, Stearylalkohol, Laurylalkohol, Myristylalkohol, Behenylalkohol, Hagebuttenöl, Sonnenblumenöl, Sojabohnenöl und Mischungen davon ausgewählt sind.

15. System nach einem vorhergehenden Anspruch, wobei die Hautpflegelösung des Weiteren von 0,1 bis 10 Gew.-% Sterol oder Sterol-Derivate enthält.

16. System nach Anspruch 15, wobei das Sterol oder Sterol-Derivat aus Cholesterin, Sitosterol, Stigmasterol, Ergosterol, Lanasterol, Sojasterol, Avocadosterolen, Cholesterinestern, Sterolestern, Lanolin und Mischungen davon ausgewählt ist.

17. System nach einem vorhergehenden Anspruch, wobei die Hautpflegelösung des Weiteren von 0,1 bis 30 Gew.-% Feuchthaltemittel enthält.

18. System nach Anspruch 17, wobei das Feuchthaltemittel aus Acetamid MEA, Aloevera-Gel, Arginin PCA, Chitosan PCA, Kupfer PCA, Maisgylceriden, Dimethylimidazolidinon, Fruktose, Glucamin, Glukose, Glukoseglutamat, Glucuronsäure, Glutaminsäure, Glycereth-7, Glycereth-12, Glycereth-20, Glycereth-26, Glycerin, Honig, hydriertem Stärkehydrolysat, hydrolysierter Maisstärke, Lactamid MEA, Milchsäure, Lactose-Lysin PCA, Mannit, Methylgluceth-10, Methylgluceth-20, PCA, PEG-2-Lactamid, PEG-10-Propylenglycol, Polyaminozucker-Kondensat, Kalium PCA, Propylenglycol, Propylenglycolcitrat, Saccharid-Hydrolysat, Saccharid-Isomerat, Natriumaspartat, Natriumlactat, Natrium PCA, Sorbit, TEA-Lactat, TEA-PCA, Harnstoff, Xylit und Mischungen davon ausgewählt ist.

**Revendications**

1. Système pour améliorer la santé cutanée d'un porteur d'articles absorbants, comprenant :

(a) un article absorbant, jetable, incluant une feuille extérieure de couverture, une doublure côté corporel, perméable aux liquides, définissant une surface faisant face au corps et connectée en relation de superposition avec la feuille extérieure de couverture ; un corps absorbant situé entre la doublure côté corporel et la feuille extérieure de couverture ; et une composition pour la santé cutanée sur au moins une portion de la surface faisant face au corps de la doublure côté corporel incluant entre 40 et 95% en poids d'émollient et entre 5 et 60% en poids de réhausseur de viscosité ; et

(b) une lingette humide incluant un substrat non-tissé et une solution pour la santé cutanée incluant entre 90 et 99% en poids de solvant hydrophile, entre 0 et 30% en poids de tensioactif et entre 0,1 et 10% en poids de principe actif d'extrait de préparation botanique et sélectionné parmi les principes actifs extraits de l'échinacée, du yucca, de l'épilobe, du thé vert, du thé noir, du thé oolong, du thé de Chine, des constituants d'extrait de thé et des mélanges de ceux-ci.

2. Système selon la revendication 1, dans lequel le principe actif d'extrait de préparation botanique est l'épicatéchine gallate ou l'épigallocatéchine 3-0-gallate.

3. Système selon la revendication 1 ou 2, dans lequel l'émollient de la composition pour la santé cutanée est sélectionné parmi la vaseline, les huiles à base végétale, les huiles minérales, la diméthicone, la lanoline, les esters de glycérol, les acides carboxyliques alcoxylés, les alcools alcoxylés, les alcools gras et les mélanges de ceux-ci.

4. Système selon l'une quelconque des revendications précédentes, dans lequel le réhausseur de viscosité de la composition pour la santé cutanée est sélectionné parmi les résines de polyoléfines, les épaississants lipophiles/ huile, les copolymères éthylène/acétate de vinyle, les argiles naturelles, les analogues synthétiques d'argiles naturelles, les argiles modifiées organiquement, les argiles modifiées quaternaires, les composés quaternaires de l'amidon, le polyéthylène, la silice, le silylate de silice, le méthylsilylate de silice, le dioxyde de silicium colloïdal, les alkyl-hydroxyéthyl-cellulose, la cire microcristalline, la cire shellac, l'hexacosanate d'hexadécyl-cosanyle, un hydroxystéarylstéarate d'alkyle en $C_{20}$-$C_{40}$, le montanate de glycol, la cire d'ozokérite, le polyperfluoro-méthylisopropyléther de la cire de lignite, le silicate de magnésium et aluminium, les polymères polyméthacrylate, les copolymères de polystyrène et les mélanges de ceux-ci.

5. Système selon l'une quelconque des revendications précédentes, dans lequel la composition pour la santé cutanée inclut, en outre, entre 5 et 55% en poids d'agent de solidification.

6. Système selon la revendication 5, dans lequel l'agent de solidification de la composition pour la santé cutanée est sélectionné parmi la cire d'abeille, le béhénate de béhényle, le benzoate de béhényle, les esters ramifiés, la cire de candelilla, la cire de carnauba, la cire de carnauba synthétique, la cire de carnauba PEG-12, la cérasine, la cire microcristalline, la cire microcristalline hydrogénée, l'hexacosanate d'hexadécylcosanyle, le polyperfluorométhylisopropyléther de la cire de lignite, les alkylméthylsiloxanes, le montanate de glycol, la cire de jojoba, la cire de lanoline, l'ozokérite, la paraffine, la paraffine synthétique, le polyéthylène, un stéarate d'(alkyle en $C_{20}$-$C_{40}$) et d'hydroxystéaryle, 1'(alkyle en $C_{30}$)diméthicone, les esters cétyliques, le stéarate de zinc, la cire shellac, l'huile de coton hydrogénée, le squalène hydrogéné, l'huile de jojoba hydrogénée et les mélanges de ceux-ci.

7. Système selon l'une quelconque des revendications précédentes, dans lequel la composition pour la santé cutanée inclut, en outre, entre 0,1 et 55% en poids de graisses ou d'huiles naturelles.

8. Système selon la revendication 7, dans lequel la graisse ou l'huile naturelle est sélectionnée parmi l'huile d'avocat, l'huile d'abricot, l'huile de babassu, l'huile de bourrache, l'huile de camélia, l'huile de canola, l'huile de ricin, l'huile de coprah, l'huile de maïs, l'huile de coton, l'huile d'onagre, l'huile de coton hydrogénée, l'huile d'amande de palmier hydrogénée, l'huile de soja maléatée, l'huile d'écume des prés, l'huile d'amande de palmier, l'huile d'arachide, l'huile de colza, l'huile de carthame, les sphingolipides, l'huile d'amande douce, le tall oil, l'acide laurique, l'acide palmitique, l'acide stéarique, l'acidelinoléique, l'alcool stéarylique, l'alcool laurylique, l'alcool myristylique, l'alcool béhénylique, l'huile de cynorhodon, l'huile de calendula, l'huile de camomille, l'huile d'eucalyptus, l'huile de genévrier, l'huile de santal, l'huile de melaleuca, l'huile de tournesol, l'huile de soja et les mélanges de ceux-ci.

9. Système selon l'une quelconque des revendications précédentes, dans lequel la composition pour la santé cutanée inclut, en outre, entre 0,1 et 10% en poids de stérols ou de dérivés de stérol.

10. Système selon la revendication 9, dans lequel le stérol ou le dérivé de stérol est sélectionné parmi le cholestérol, le sitostérol, le stigmastérol, et l'ergostérol, de même que les esters de cholestérol/lanostérol en $C_{10}$-$C_{30}$, le cholecalciférol, l'hydroxystéarate de cholestéryle, l'isostéarate de cholestéryle, le stéarate de cholestéryle, le 7-dihydrocholestérol, le dihydrocholestérol, l'octyldécanoate de dihydrocholestéryle, le dihydrolanostérol, l'octyldécanoate de dihydrolanostéryle, l'ergocalciférol, le stérol de tall oil, l'acétate de stérol de soja, le lanostérol, le stérol de soja, les stérols d'avocat, les esters de stérol et les mélanges de ceux-ci.

11. Système selon l'une quelconque des revendications précédentes, dans lequel le tensioactif de la solution pour la santé cutanée a une gamme HLB allant de 6 à environ 18.

12. Système selon la revendication 11, dans lequel le tensioactif de la solution pour la santé cutanée est sélectionné parmi la cire émulsifiante NF, le stéarate de glycéryle, le stéarate de glycéryle SE, le stéarate de glycol, le stéarate de glycol SE, le stéarate de glycereth-20, le béhénate de glycéryle, l'hydroxystérarate de glycéryle, le laurate de glycéryle SE, l'oléate de glycéryle, l'oléate de glycéryle SE, l'oléate de propylène glycol, l'oléate de propylène glycol SE, le stéarate de propylène glycol, le stéarate de propylène glycol SE, le stéarate de sorbitan, le trioléate de sorbitan, le copolyol de diméthicone, le cocamphoacétate de sodium, le lauroamphodiacétate disodique, le caprylamphodipropionate disodique, la cocamidopropyl-bétaïne, la cluramidopropylbétaïne, l'octyl-bétaïne, la cocamidopropyl hydroxysultaïne, le laurylsulfosuccinate de diammonium, le diméthicone copolyol sulfosuccinate disodique, le laurethsulfosuccinate de diammonium, le laurylsulfate de sodium, le laurethsulfate de sodium, le laurylsulfate d'ammonium, le laurylsulfate de TEA, le polyoxyéthylène/huile de ricin hydrogénée et les mélanges de ceux-ci.

13. Système selon l'une quelconque des revendications précédentes, dans lequel la solution pour la santé cutanée inclut, en outre, entre 0,1 et 30% en poids de graisses ou d'huiles naturelles.

14. Système selon la revendication 13, dans lequel les graisses ou les huiles naturelles sont sélectionnées parmi l'huile d'avocat, l'huile d'abricot, l'huile de babassu, l'huile de bourrache, l'huile de camélia, l'huile de canola, l'huile de ricin, l'huile de coprah, l'huile de maïs, l'huile de coton, l'huile d'onagre, l'huile de coton hydrogénée, l'huile d'amande de palmier hydrogénée, l'huile de soja maléatée, l'huile d'écume des prés, l'huile d'amande de palmier, les phospholipides, l'huile de colza, l'acide palmitique, l'acide stéarique, l'acide linoléique, l'alcool stéarylique, l'alcool laurylique, l'alcool myristylique, l'alcool béhénylique, l'huile de cynorhodon, l'huile de tournesol, l'huile de soja et les mélanges de ceux-ci.

15. Système selon l'une quelconque des revendications précédentes, dans lequel la solution pour la santé cutanée inclut, en outre, entre 0,1 et 10% en poids de stérol ou de dérivé de stérol.

16. Système selon la revendication 15, dans lequel le stérol ou le dérivé de stérol est sélectionné parmi le cholestérol, le sitostérol, le stigmastérol, l'ergostérol, le lanostérol, le stérol de soja, les stérols d'avocat, les esters de cholestérol, les esters de stérol, la lanoline et les mélanges de ceux-ci.

17. Système selon l'une quelconque des revendications précédentes, dans lequel la solution pour la santé cutanée inclut, en outre, entre 0,1 et 30% en poids d'humectant.

18. Système selon la revendication 17, dans lequel l'humectant est sélectionné parmi l'acétamide MEA, le gel d'aloe vera, l'arginine PCA, le chitosan PCA, le cuivre PCA, les glycérides de maïs, la diméthy-imidazolidinone, le fructose, la glucamine, le glucose, le glutamate de glucose, l'acide glucuronique, l'acide glutamique, le glycereth-7, le glycereth-12, le glycereth-20, le glycereth-26, la glycérine, le miel, le miel hydrogéné, l'hydrolysat d'amidon hydrogéné, l'amidon de maïs hydrolysé, le lactamide MEA, l'acide lactique, le lactose lysine PCA, le mannitol, le méthyl gluceth-10, le méthyl gluceth-20, le PCA, le lactamide PEG-2, le propylène glycol PEG-10, le condensat de sucre polyamino, le PCA potassique, le propylène glycol, le citrate de propylène glycol, l'hydrolysat de saccharide, l'isomérat de saccharide, l'aspartate de sodium, le lactate de sodium, le PCA sodique, le sorbitol, le lactate TEA, le TEA-PCA, l'urée, le xylitol et les mélanges de ceux-ci.

**FIG. 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4798603 A
- US 5176668 A
- US 5176672 A
- US 5192606 A
- US 5496298 A
- US 5509915 A
- US 6217890 B
- US 5482765 A
- US 5879341 A
- US 5843056 A
- US 6309736 B
- US 5486166 A
- US 5490846 A
- US 5364382 A
- US 6149934 A
- US 4100324 A
- US 4604313 A
- US 5350624 A